# EUROPEAN PATENT APPLICATION

(11) **EP 2 363 390 A1**
(43) Date of publication of application: **07.09.2011**
(21) Application number: 09824864.4
(22) Date of filing: 02.11.2009
(51) Int. Cl.: C07D 207/273, A61K 31/593, A61P 3/02, A61P 11/06, A61P 19/08, A61P 19/10

(54) **DERIVED LACTAM FROM D3 VITAMIN**

(30) Priority: 04.11.2008 JP 2008283162
(71) Applicant: Teijin Pharma Limited, Tokyo 100-0013 (JP)
(72) Inventor: NAKAMURA, Yuko, Hino-shi Tokyo 191-0065 (JP); SAITO, Hiroshi, Hino-shi Tokyo 191-0065 (JP); NAGASAWA, Kazuo, Koganei-shi Tokyo 184-0012 (JP); KITTAKA, Atsushi, Sagamihara-shi Kanagawa 229-0106 (JP)
(74) Representative: Hallybone, Huw George
(86) International application number: PCT/JP2009/069018
(87) International publication number: WO 2010/053165

(57) **Abstract**

Compound represented by formula (1) or a pharmaceutically acceptable solvate thereof, usefull for treating or preventing Paget's disease of bone, hypercalcmia, osteoporosis or asthma. (1) R¹ represents a C₁-C₆alkyl group or C₇-C₁₅ aralkyl group (the aromatic ring of which can be substituted by a C₁-C₈ alkyl group, C₁-C₆ alkoxy group, a hydroxyl group, a halogenatom or a trifluoromethyl group), R² represents a C₁-C₆ alkyl group, and R³ represents a C₁-C₆ alkyl or alkoxy group, which can be substituted with a hydroxyl group.

## Description

### TECHNICAL FIELD

The present invention relates to vitamin D₃ lactam derivatives, which are useful as pharmaceutical agents or pharmaceutically acceptable solvates thereof. More specifically, it relates to vitamin D₃ lactam derivatives which are useful for treating and /or preventing one or plurality of diseases selected from osteoporosis, hypercalcaemia, Paget's disease of bone, and asthma, or pharmaceutically acceptable solvates thereof.

### BACKGROUND OF THE INVENTION

Paget's disease of bone is defined as a disease of uncertain origin characterized by symptoms such as bone deformity and ostealgia resulting from aberrant acceleration in bone resorption such as in the pelvis, femur, and cranial bones. The current therapeutic agents for Paget's disease of bone include, bisphosphonate preparations and calcitonin preparations which have also been used as therapeutic agents for osteoporosis. The former preparations are required at 4 to 5 times higher dosage for the patients with Paget's disease of bone than for patients with osteoporosis, therefore compliance remains an issue, and the latter preparations have the drawback that they cannot attain sufficient anti-resorptive activity. Furthermore, the complete cure of the disease cannot be expected because these preparations are palliative treatments based on the anti-resorptive activity of the agents.

In recent years, it has been shown that osteoclast precursors and osteoclasts obtained from patients with Paget's disease of bone possess 1α,25-dihydroxyvitamin D₃ receptors (see reference 1) and that the susceptibility to 1α,25-dihydroxyvitamin D₃ is 10 to 100 times higher than that of the osteoclast precursors from normal individuals (see reference 2). Furthermore, since the blood levels of 1α,25-dihydroxyvitamin D₃ in the patients with Paget's disease of bone are equivalent to that of normal individuals, it has been suggested that the acceleration of bone resorption by endogenous 1α,25-dihydroxyvitamin D₃ plays an important role in the onset of Paget's disease of bone. Accordingly, the compounds that inhibit the action of 1α=25-dihydroxyvitamin D₃ on osteoclast precursors and osteoclasts, i.e., the compounds such as a vitamin D₃ receptor-antagonist can ultimately inhibit accelerated bone resorption in patients with Paget's disease of bone, so that such compounds are expected to provide a therapeutic effect superior to the current anti-resorptive agents and also prevent the onset of the disease.

On the contrary, the elevation of the production of vitamin D₃ in various diseases, for example, such as lymphoma (see reference 3), tuberculosis (see reference 4), sarcoidosis (see reference 5), candidiasis (see reference 6), granuloma (see reference 7), lepra (see reference 8), primary hyperparathyroidism, and malignant tumor develop hypercalcaemia. Since it has been known that blood calcium levels increase by the action of an active form of vitamin D₃, compounds being antagonistic to the active form of vitamin D₃, more specifically, a vitamin D₃ receptor-antagonist is thought to be useful for treating and/or preventing hypercalcaemia.

Furthermore, the vitamin D₃ receptor-antagonist is also thought to be useful as a therapeutic agent for osteoporosis. In this disease, bone quantity decreases as a result of bone resorption exceeding bone formation. In most cases, the disease develops in post-menopausal stage or with aging. Bisphosphonates, vitamin D₃ derivatives, estrogen, calcitonin and the like are used as therapeutic agents for this disease. In recent years, moreover, parathyroid hormone (PTH) formulation having an unprecedentedly strong osteogenesis promotion activity has emerged in a clinical stage, and a more effective medication has become available. However, because the PTH formulation is a parenteral injection, there are problems such as convenience, compliance, costs and the like. Therefore, if oral preparations having the same action equivalent to the PTH action can be achieved at low cost, they can be useful drugs. Meanwhile, PTH secretion is controlled by blood calcium and 1α,25-dihydroxyvitamin D₃, an active form of vitamin D₃, so that as the concentration of such compounds decrease, the quantity of PTH secretion increases. Therefore, compounds being antagonistic to 1α,25-dihydroxyvitamin D₃, i.e., a vitamin D₃ receptor-antagonist can be expected to promote PTH secretion and to have a therapeutic and/or prophylactic effect similar to the PTH formulation mentioned above.

Additionally, in recent years, it has been shown that 1α,25-dihydroxyvitamin D₃ receptor-deficient mice do not develop pathological conditions of allergic asthma compared to normal mice (see reference 9). This suggests that the vitamin D₃ receptor-antagonist, which inhibits the action of vitamin D₃, is useful as a therapeutic and/or prophylactic agent for asthma.

Meanwhile, in the abstract (published on November 5, 2002) at the 22nd Medicinal Chemistry Symposium & the 11th Annual Meeting of the Division of Medicinal Chemistry in the Pharmaceutical Society of Japan, compounds represented in Formula (1) hereinafter described, in which R¹= R² = Me, and R¹=Bn and R²= Me were disclosed. However, these compounds are not included in the scope of the present invention. In addition, there was no suggestion on the vitamin D₃ receptor-antagonist activity in this disclosure.

In addition, a compound represented in Formula (1) hereinafter described, in which R³ is hydrogen atom, was disclosed in the Patent document 1. However, this compound is not included in the scope of the present invention.

In addition, a vitamin D₃ derivative having a lactam structure of the side chain thereon was disclosed in the Patent document 2. The chemical structure of this compound, however, is different from the compounds of the present invention, because nitrogen atom in the lactam ring is not substituted and substituents at the 25-position do not include a hydroxyl group. There was also no suggestion on the vitamin D₃ receptor-antagonist activity in this disclosure.

Compounds described in the Patent document 3, Patent document 4, Patent document 5, Patent document 6, Patent document 7, and Patent document 8 are known as vitamin D₃ derivatives having the vitamin D₃ receptor-antagonist activity. However, since the compounds described in the former three have α-exomethylenelactone structure on the side chain, and the compounds described in the latter three have 22-ene-24-hidroxy structures on the side chain, their chemical structures are apparently different from the compounds of the present invention.

Additionally, the vitamin D₃ derivative having the vitamin D₃ receptor-antagonist activity was disclosed in reference 9, but this compound is different from the compound of the present invention since this compound has an ester group bound at the 1-position.

It has been known that vitamin D₃ derivatives have a broad range of interesting physiological activities, and a wide variety of vitamin D₃ derivatives have been synthesized by a number of researchers. Nevertheless, the vitamin D₃ receptor-antagonist activity has been observed in only these four groups of derivatives belonging to a narrow range having nothing in common. More specifically, it can be said that there is no accumulated knowledge on the relationship between the chemical structures of vitamin D₃ derivatives and the vitamin D₃ receptor-antagonist activity. Even though the vitamin D₃ receptor-antagonist activity is found in a certain vitamin D₃ compound, a compound with a slight modification in the chemical structure of the original compound cannot be expected to have the vitamin D₃ receptor-antagonist activity.
Patent document, 1. Description of International Publication WO05/42482
Patent document 2: Description of International Publication WO00/24712
Patent document 3: Description of International Publication WO95/33716
Patent document 4: Description of International Publication WO03/70716
Patent document 5: Description of International Publication WO04/67525
Patent document 6: Description of International Publication WO94/07853
Patent document 7: Description of International Publication WO97/00242
Patent document 8: Description of International Publication WO97/41096
Patent document 9: Description of International Publication WO03/00634
Reference 1: Bone, 18:295-299, 1996.
Reference 2: J. Bone Miner. Res., 15:228-2.36, 2000.
Reference 3: Blood, 82:1383-1394, 1993.
Reference 4: N. Engl. J. Med., 311:1683-1685, 1984.
Reference 5: J. Clin. Endocrinol. Metab., 60:960-966, 1985.
Reference 6: Am. J. Med., 74:721-724, 1983.
Reference 7: Am. J. Nephrol., 13:275-277, 1993.
Reference 8: Ann. Intern. Med., 105:890-891, 1986.
Reference 9: J. Immunol., 173:3432-3436, 2004.

### DISCLOSURE OF THE INVENTION

An object of the present invention is to provide compounds that antagonisze the action of the active form of vitamin D₃, i.e., vitamin D₃ receptor antagonists. Such a vitamin D₃ receptor antagonist is considered useful as an active ingredient of a therapeutic and/or prophylactic agent for Paget's disease of bone, hypercalcaemia, osteoporosis, and asthma.

The inventors of the present invention reached the present invention indicated below resulting from the study with the above object,

Accordingly, the present invention is a vitamin D₃ derivative represented in Formula (1) or a pharmaceutically acceptable solvate thereof.

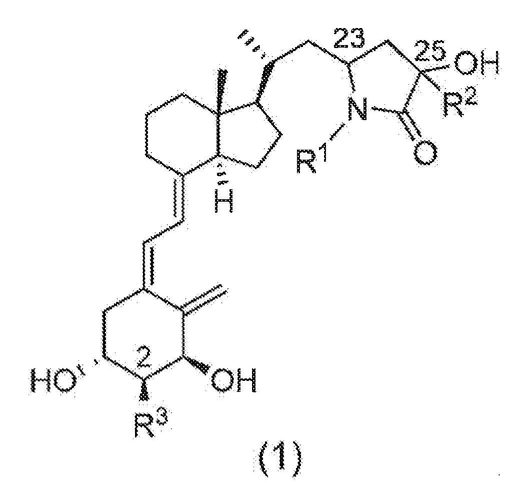

In the formula (1), R¹ represents a C₁₋C₁₀ alkyl group, or C₇-C₁₅ aralkyl group whose aromatic ring is optionally substituted with a C₁-C₆ alkyl group, a C₁-C₆ alkoxy group, a hydroxyl group, a hologen atom, or a trifluoromethyl group; R² represents a C₁-C₆ alkyl group; R³ represents a C₁-C₆ alkyl group optionally substituted with a hydroxyl group or a C₁-C₆ alkoxy group optionally substituted with a hydroxyl group. Configurations of the 23-position and the 25-position can be either of (S) and (R) configurations, independently,

Further, this invention is a therapeutic and/or prophylactic agent comprising the vitamin D₃ derivatives represented in Formula (1) above or a pharmaceutically acceptable solvates thereof, as an active ingredient, for one or plurality of diseases selected from the group consisting of Paget's disease of bone, hypercalcaemia, osteoporosis and asthma.

According to the present invention, compounds that antagonize the action of active form of vitamin D₃, i.e., a vitamin D₃ receptor-antagonist is provided. Such a vitamin D₃ receptor-antagonist is used as an active ingredient of a therapeutic and/or preventive agent for Paget's disease of bone, hypercalcaemia, osteoporosis and asthma.

### BEST MODE FOR CARRYING OUT THE INVENTION

The terms of the present invention are defined in the following.

An alkyl group refers to a linear, branched chain or cyclic aliphatic hydrocarbon group. A C₁-C₁₀ alkyl group means an alkyl group having 1 to 10 carbon atom(s), and for example, a methyl group, an ethyl group, a propyl group, an isopropyl group, a butyl group, an isobutyl group, a pentyl group, an isopentyl group, a hexyl group, an octyl group, a decyl group, a cyclopropyl group, a cyclopropylmethyl group, a cyclohexyl group are cited as specific groups. Similarly, a C₁-C₆ alkyl group means an alkyl group having 1 to 6 carbon atom(s) such as a methyl group, an ethyl group, a propyl group, an isopropyl group, a butyl group, an isobutyl group, a pentyl group, an isopentyl group, a hexyl group, a cyclopropyl group, a cyclopropylmethyl group, a cyclohexyl group.

A C₁-C₆ alkoxy group refers to a linear, branched or cyclic aliphatic hydrocarbonoxy group having 1 to 6 carbon atom(s). For example, a methoxy group, an ethoxy group, a propoxy group, an isopropoxy group, a butoxy group, an isobutoxy group, a pentyloxy group, an isopentyloxy group, a hexyloxy group, a cyclopropoxy group, a cyclopropylmethoxy group, a cyclohexyloxy group are examples of specific groups.

A C₇-C₅ aralkyl group refers to a linear, branched or cyclic aliphatic hydrocarbon group substituted with an aromatic hydrocarbon group, having 7 to 15 carbon atoms in total. For example, a benzyl group, a phenethyl group, a 3-phenylpropyl group, a 4-phenylbutyl group, a 5-phenylpentyl group, a 1-naphthylmethyl group, a 2-naphthylmethyl group, a 2-(1-naphthyl)ethyl group, a 2-(2-naphthyl)ethyl group, a 3-(1-naphthyl)propyl group, a 3-(2-naphthyl)propyl group, a 4-(1-naphthyl)butyl group, a 4-(2-naphthyl)butyl group, a 5-(1-naphthyl)pentyl group, a 5-(2-naphthyl)pentyl group are examples of specific groups.

In the above Formula (1), R¹ represents a C₁-C₁₀ alkyl group, or a C₇-C₁₅ aralkyl group whose aromatic ring is optionally substituted with a C₁-C₆ alkyl group, a C₁-C₆ alkoxy group, a hydroxyl group, a halogen atom, or a trifluoromethyl group. Among them, a phenethyl group, a 2-naphthylmethyl group, a 3,5-dimethoxybenzyl group, a 3,5-diethoxybenzyl group, a 3,5-dipropoxybenzyl group are preferred, and a phenethyl group, a 3,5-diethoxybenzyl group, a 2-naphthylethyl group are particularly preferred.

In the above Formula (1), R² is a C₁-C₆ alkyl group. Among them, a methyl group and an ethyl group are preferred; a methyl group is particularly preferred.

In the above Formula (1), R³ is a C₁-C₆ alkyl group optionally substituted with a hydroxyl group, or a C₁-C₆ alkoxy group optionally substituted with a hydroxyl group. Among them, a methyl group, a 3-hydroxypropyl group, a 3-hydroxypropoxy group are particularly preferred.

In the above Formula (1), the configurations of the 23-position and the 25-position may be independently the configurations of (S) or (R); an isomer with the configuration of 23(S) and 25(S) is particularly preferred.

Preferable specific examples of vitamin D₃ derivatives represented in Formula (1) of the present invention are shown in Table 1. The configurations of the 23-position and 25-position of the compounds shown in the table, may be either the configurations of (S) or (R) unless otherwise specified.

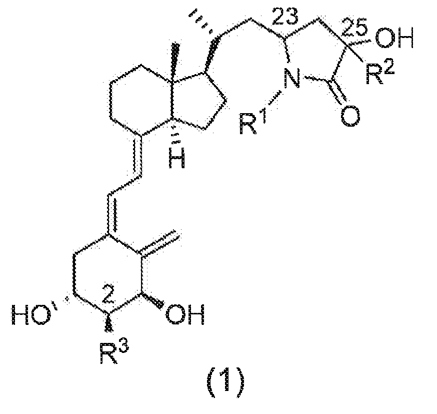

**Table 1**

| Compound No. | R¹ | R² | R³ |
|---|---|---|---|
| 11 | phenethyl | methyl | methyl |
| 12 | phenethyl | methyl | 3-hydroxypropyl |
| 13 | phenethyl | methyl | 3-hydroxypropoxy |
| 21 | 2-naphthylmethyl | methyl | methyl |
| 22 | 2-naphthylmethyl | methyl | 3-hydroxypropyl |
| 23 | 2-naphthylmethyl | methyl | 3-hydroxypropoxy |
| 31 | 3,5-dimetlioxybenzyl | methyl | methyl |
| 32 | 3,5-dimethoxybenzyl | methyl | 3-hydroxypropyl |
| 33 | 3,5-dimethoxybenzyl | methyl | 3-hydroxypropoxy |
| 41 | 3,5-diethoxybenzyl | methyl | methy |
| 42 | 3,5-diethoxybenzyl | methyl | 3-hydroxypropyl |
| 43 | 3,5-diethoxybenzyl | methyl | 3-hydroxypropoxy |
| 51 | 3,5-dipropoxybenzyl | methyl | methyl |
| 52 | 3,5-dipropoxybenzyl | methyl | 3-hydroxypropyl |
| 53 | 3,5-dipropoxybenzyl | methyl | 3-hydroxypropoxy |
| 61 | 2-naphthylethyl | methyl | methyl |
| 62 | 2-naphthylethyl | methyl | 3-hydroxypropyl |
| 63 | 2-naphthylethyl | methyl | 3-hydroxypropoxy |

Vitamin D₃ derivatives represented in Formula (1) may be produced by any method. However, it can be produced, for example, as described in Scheme 1. Accordingly, compound (2) obtained from vitamin D₂ by a known method (e.g., the description of the International Publication WO95/33716) and an R¹-bound hydroxylamine are allowed to react in the presence of triethylamine to produce a nitrone (3). To the nitrone is added R² -substituted acrylic ester (4) to obtain an isoxazolidine derivative (5). In the case where R is not a methyl group, the R is converted to a methyl group, the N-O bond then is reduced, and a hydroxyl group on the lactam ring is protected with a TMS group, to obtain a bromomethylene derivative having a lactam ring (6). According to a method of Trost et at, (J. Am. Chem. Soc. 114: 9836-9845, 1992), the obtained compound above is subjected to a coupling reaction with enyne compound (7) (in the absence of a hydroxyl group R^{3a} has the same definition of R³, in the presence of a hydroxyl group R^{3a} means R³ which is protected by a proper protecting group) in the presence of a palladium catalyst, subsequently TMS and TBS groups are removed for the deprotection to obtain the objective compound (1). The enyne compound (7) can be obtained in the art referring to a known method. For example, the method by Konno et al. is found in J. Med. Chem. (43:427-4265, 2000) in the case where R^{3a} is a methyl group, the method by Suhara et al. is found in J. Org. Chem. (66:8760-8771, 2001) in the case where R^{3a} is an ethyl group, a propyl group, a butyl group, a *t-*butyldimethylsilyloxymethyl group, a 2-*t*-butyldimethylsilyloxyethyl group, a 3-*t-*butyldimethylsilyloxypropyl group, and 4-*t-*butyldimethylsilyloxybutyl group, the method by Kittaka et al. is found in Org. Lett. (2:2619-2622, 2000) in the case where R^{3a} is 2-*t-*butyldimethylsilyloxyethoxy group, a 3-*t*-butyldimethylsilyloxypropoxy group, and a 4-*t-*butyldimethylsilyloxybutoxy group.

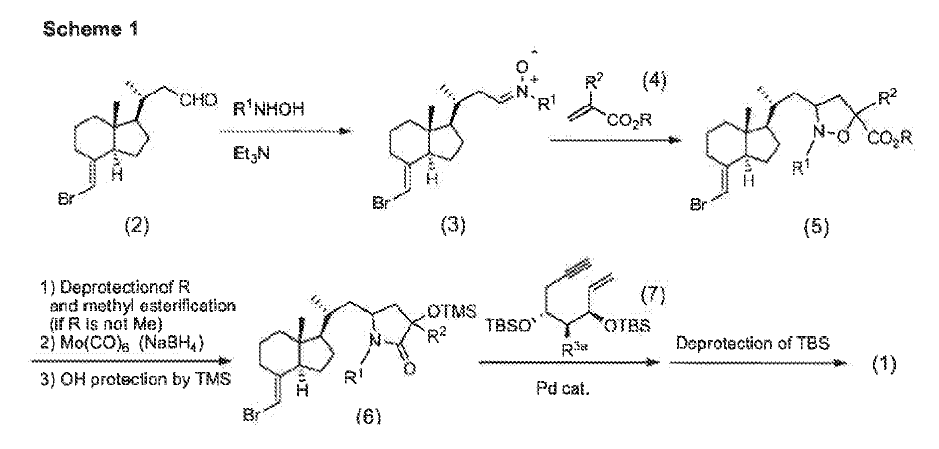

The vitamin D₃ derivatives obtained as described above can be converted to pharmaceutically acceptable solvates mentioned above if desired. Such solvents are water, methanol, ethanol, 1-propanol, 2-propanol, butanol, 2-methyh-2-propanol, acetonitrile, acetone, methyl ethyl ketone, chloroform, ethyl acetate, diethyl ether, *t-*butyl methyl ether, benzene, toluene, DMF, DMSO and the like. Water, methanol, ethanol, 1-propanol, 2-propanol, acetonitrile, acetone, methyl ethyl ketone, and ethyl acetate are preferred.

The compounds in the present invention can be administered, as a pharmaceutical active ingredient by performing conventional pharmaceutical formulation, orally, or parenterally such as intravenously, subcutaneously, intramuscularly, percutancously, intranasally or intrarectally, or by inhalation. While the dosage of the active ingredient of the present invention will vary depending upon the administration routes, the age, the sex and condition of a patient, generally a therapeutically effective dose of the active ingredient of the compounds of the present invention is between 0.01 and 10000 µg per day, more preferably is between 0.1 and 1000 µg per day, and the frequency of the administration is generally 1 to 3 times per day. Preferably, pharmaceutical formulations to fulfill such conditions are prepared.

### EXAMPLES

Hereinafter, the present invention is further explained in detail by examples, but the prevent invention is not limited by them. The compound number in each example corresponds to the compound number in the aforementioned Table or those in the aforementioned Scheme 1.

### Reference Example 1

### Production of 2-phenylethylhydroxylamine

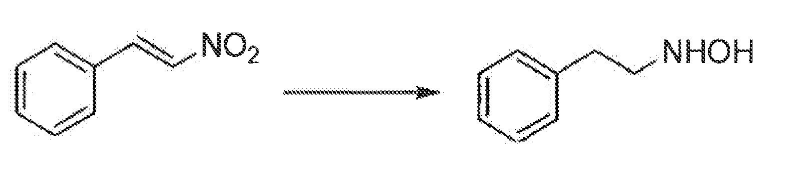

To a solution of 1.0 M BH₃-THF in THF (1.1 mL, 1.1 mmol) was added dropwise a solution of *trans*-β-nitrostyrene (160.0 mg, 1.1 mmol) in THF (2.2 mL) at 0°C under nitrogen. NaBH₄ (3.3 mg, 0,087 mmol) was added to the mixture and the resultant solusion was stirred at room temperature for 20 minutes. After adding water (5 mL) to the mixture at 0°C, the resultant mixture was acidified by the addition of 2 M HCl aqueous solution, and the mixture was stirred at 65°C for 4 hours. After extracting the mixture with ethyl acetate, 15% NaOH aqueous solution and NaCl were added to the aqueous layer, and the aqueous layer was further extracted with ethyl acetate. The organic layer was dried over anhydrous magnesium sulfate, and the solvent was evaporated after filtration. The residue was purified by flash column chromatography (chloroform:methanol =1:0 → 10:1) to afford the objective compound as white crystals (62.7 mg, 43%).
¹H-NMR (CDCl₃) δ: 7.47 (brs, 1H), 7.31-7.20 (m, 5H), 3.26 (t, J = 7.3 Hz, 2H), 2.97 (t, J = 7.3 Hz, 2H).

### Reference Example 2

### Production of 3,5-diethoxybenzylhydroxylamine

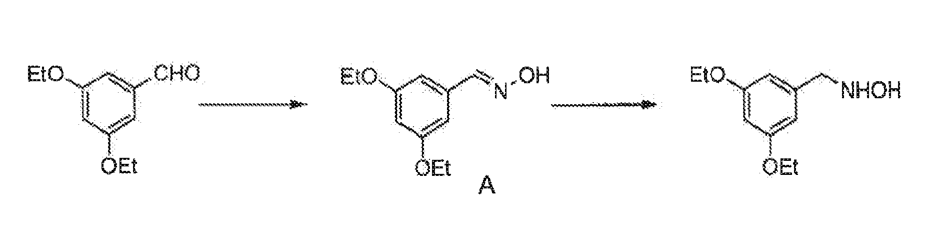

(1) To a solution of 3,5-diethoxybenzaldehyde (1.08 g, 5.56 mmol) in dichloromethane (56.0 mL) was added hydroxylamine hydrochloride (773 mg, 11.12 mmol) and Et₃N (3.1 mL, 22.24 mmol) and the mixture was stirred at room temperature for 2 hours. Saturated sodium bicarbonate solution was added to the mixture and the resultant mixture was extracted with dichloromethane. The organic layer was dried over anhydrous magnesium sulfate and the solvent was evaporated after filtration. The residue was purified by flash column chromatography (hexane:ethyl acetate = 10:1) to afford compound A (1.05 g, 90 %).
   ¹H-NMR (CDCl₃) δ: 8.05 (s, 1H), 6.71 (d, J = 2.4Hz, 2H), 6.48 (t, J = 2.2 Hz, 1H), 4.02 (q, J = 7.0 Hz, 4H), 1.40 (t, J = 7.0Hz, 6H).
(2) To a solution of the compound A (960 mg, 4.59 mmol) in methanol (46.0 mL) was added NaBH₃CN (201.8 mg, 3.21 mmol) at room temperature. 3 M HCl aqueous solution was added to the mixture and the resultant mixture was stirred at room temperature for 4.5 hours while maintaining pH at 3. NaBH₃CN (90.0 mg, 1.43 mmol) was further added to the mixture and the resultant mixture was further stirred for 0.5 hours. The mixture was alkalized by the addition of 15% NaOH aqueous solution at 0°C, and then the mixture was extracted with ethyl acetate. The organic layer was dried over anhydrous magnesium sulfate and the solvent was evaporated after filtration. The residue was purified by flash column chromatography (n-hexane:ethyl acetate =1:1) to afford 3, 5-diethoxybenzylhydroxylamine as a clear and colorless oily product. (673.9 mg, 70 %).
   ¹H-NMR (CDCl₃) δ: 6.44 (d, J = 2.2 Hz, 2H), 6.37 (t, J = 2.2 Hz, 1H), 6.11 (brs, 1H), 3.98 (q, J = 7.0 Hz, 4H), 3.90 (s, 2H), 1.39 (t, J = 7.0Hz, 6H).

### Reference Example 3

### Production of 2-naphthylethylhydroxylamine

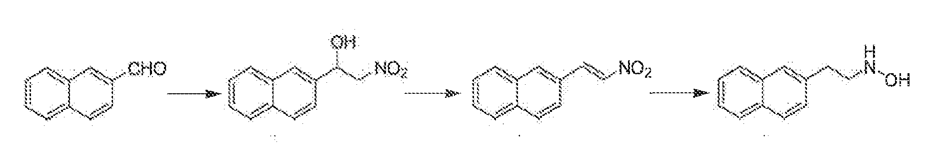

(1) To a solution of triethylamine (10 mL, 71.60 mmol) was added 2-naphthaldehyde (600 mg, 3.84 mmol) and nitromethane (0.83 mL, 15.36 mmol), and the mixture was stirred at room temperature for 29 hours under nitrogen. Water was added to the mixture and the resulting mixture was extracted with ethyl acetate. The organic layer was dried over anhydrous magnesium sulfate and the solvent was evaporated after filtration. The residue was purified by column chromatography on silica gel (hexane:ethyl acetate = 20:1 → 4:1) to afford a compound B (548 mg, 66 %).
(2) The compound B (548 mg, 2.52 mmol) was dissolved in dichloromethane (10 mL) and triethylamine (12.4 mL, 89 mmol) was added thereto at 0°C. Methanesulfonyl chloride (273 µL, 3.51 mmol) was added dropwise to the mixture, and the resultant mixture was stirred for 25 minutes under nitrogen. Water was added to the mixture and the resultant mixture was extracted with dichloromethane. The organic layer was dried over anhydrous magnesium sulfate and the solvent was evaporated after filtration. The residue was purified by column chromatography on cilica gel (hexane:ethyl acetate = 50:1→10:1) to afford compound C (440 mg, 94%).
(3) Under nitrogen, to a solution of 1.1 M BH₃-THF in THF (8.43 mL, 9.174 mmol) was added dropwise a solution of the compound C (1872.5 mg, 9.174 mmol) in THF (20 mL) at 0°C. NaBH₄ (30 mg, 0.793 mmol) was added and the mixture was stirred at room temperature for 18.5 hours. After the addition of water at 0°C, the mixture was acidified by the addition of 3 M HCl aqueous solution, and the resulting mixture was stirred at 65°C for 12 hours. After the extraction of the mixture with ethyl acetate, 15% NaOH aqueous solution and NaCl were added to the aqueous layer, and the aqueous layer was further extracted with ethyl acetate. The organic layer was dried over anhydrous magnesium sulfate and the solvent was evaporated after filtration. The residue was purified by column chromatography on silica gel (chloroform:methanol::: 1:0 → 9:1 → 5:1) to afford the objective compound D (880 mg, 51 %).
   ¹H-NMR (CDCl₃) δ: 7.84-7.03 (m, 7H), 5.92 (brs, 2H), 3.29 (t, J = 5.82 Hz, 2H), 3.08 (t, J = 5.85 Hz, 2H).

### Example 1

### Production of 2α-methyl-1α,25-dihidroxyvitamin D₃-26,23-lactam-N-(2-phenethyl) (compound No. 11)

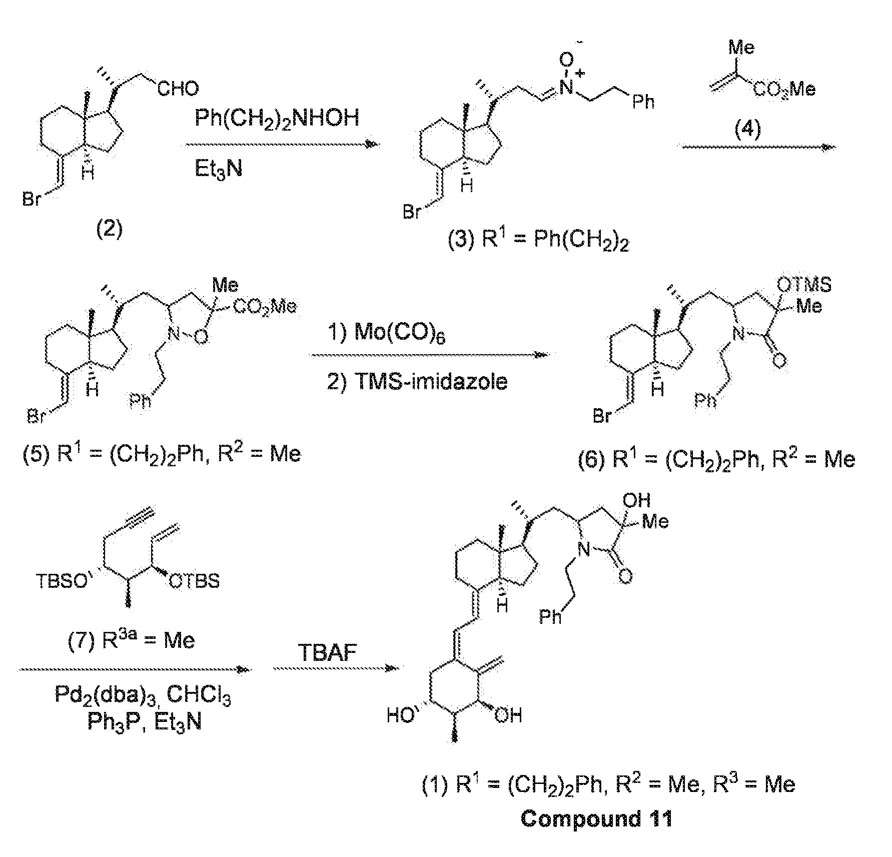

(1) To a dichloromethane (40 mL) solution of compound (2) (1.67 g, 5.57 mmol), which is obtained by the method described in literature (e.g., the description of the International Publication WO95/33716), was added 2-phenethylhydroxylamine (1.06 g, 7.70 mmol), which is obtained in the Reference Example 1, and Et₃N (10 mL, 71.7 mmol), and the mixture was stirred at room temperature for 2 hours under nitrogen. The reaction solution was cooled to 0°C, and a saturated ammonium chloride aqueous solution was added to the solution, and the resulting mixture was extracted with dichloromethane. The organic layer was dried over anhydrous sodium sulfate and the solvent was evaporated after filtration.
(2) To a solution of the resulting residue in toluene (40 mL) was added methyl methacrylate (compound (4) (R² = R = Me), 3.0 mL, 27.9 mmol) and the mixture was stirred at 90°C for 4 hours under nitrogen. After cooling the mixture to room temperature, the solvent was evaporated, and the residue was purified by flash column chromatography (hexane:ethyl acetate = 8:1) to afford compound (5) (R¹ = (CH₂)₂Ph, R² = R = Me) as a clear and colorless oily product (3.04 g, 100%).
   ¹H-NMR (CDCl₃) δ: 7.31.-7.17 (m, 5H), 5.65 (brs, 1H), 3.79-3.76 (m, 3H), 3.15-2.70 (m, 5H), 2.06-1.20 (m, 20H), 0.96-0.90 (m, 3H), 0.57-0.51 (m, 3H).
(3) The resulting compound (5) (R¹ = (CH₂)₂Ph, R² = R = Me) (3.04 g, 5.85 mmol) was dissolved in a mixed solvent of acetonitrile (35 mL) and water (5 mL), molybdenum hexacarbonyl (2.4 g, 9.07 mmol) was added to the mixture, and the resulting mixture was stirred at 80°C for 19 hours. After cooling the reaction mixture to room temperature, the mixture was filtered on Celite and concentrated under reduced pressure. The resulting residue was purified by column chromatography on silica gel (n-hexane:ethyl acetate = 2:1) to afford a lactam reduction product as a white solid (1.58 g, 55%).
   ¹H-NMR (CDCl₃) δ: 7.34-7.17 (m, 5H), 5.68-5.64 (m, 1H), 4.03-3.93 (m, 1H), 3.89-3.75 (m, 1H), 3.53-3.40 (m, 1H), 3.30-3.13 (m, 2H), 2.95-2.61 (m, 4H), 2.40-2.10 (m, 2H), 2.05-0.85 (m, 17H), 0.60-0.55 (m, 3H).
(4) The resulting lactam reduction product (1.58 g. 3.23 mmol) was dissolved in 50 mL of anhydrous dichloromethane, trimethylsilylimidazole (2.4 mL, 16.15 mmol) was added, and the mixture was stirred at room temperature for 18 hours under nitrogen. After adding water to the reaction mixture with stirring at 0°C, the mixture was extracted with dichloromethane, and the organic layer was dried over anhydrous sodium sulfate. The resulting residue concentrated under reduced pressure was purified by column chromatography on silica gel (n-hexane:ethyl acetate = 4:1) to afford compound (6) (R¹ = Ph (CH₂)₂, R² = Me) as a pale yellow oily product (1.89 g, 100%).
   ¹H-NMR(CDCl₃) δ: 7.32m7.17 (m, 5H), 5.68-5.63 (m, 1H), 3.97-3.86 (m, 1H), 3.83-3.67 (m, 1H), 3.55-3.46 (m, 1H), 3.23-3.09 (m, 2H), 2.95-2.69 (m, 4H), 2.27 (m, 1H), 2.12-0.85 (m, 18H), 0.59-0.55 (m, 3H), 0.15 (s, 9H).
(5) Triphenylphosphine (112 mg, 0.428 mmol) and tris(dibenzylideneacetone) dipaliadium (0)-chloroform adduct (55 mg, 0.054 mmol) were dissolved in a mixed solution of anhydrous toluene (4 mL) and triethylamine (4 mL), and the mixture was stirred at room temperature for 10 minutes. To the mixture was added the resulting compound (6) (R¹ = (CH₂)₂ Ph, R² = Me) (200 mg, 0.357 mmol) and a solution of compound (7) (R^{3a} = Me) (164 mg, 0.428 mmol) which can be obtained by the method described in literature (e.g., J. Med. Chem., 43: 4247-4265, 2000) in anhydrous toluene (4 mL) and the mixture was stirred at 120°C for 2 hours under argon. To the reaction solution was added a saturated ammonium chloride aqueous solution and the reaction solution was washed with ethyl acetate. After adjusting the aqueous layer to weak basic by the addition of saturated sodium bicarbonate aqueous solution, the aqueous layer was extracted with ethyl acetate. The combined organic layer was washed with saturated brine and was dried over anhydrous sodium sulfate. The resulting residue concentrated under reduced pressure was purified crudely by column chromatography on silica gel (n-hexane:ethyl acetate = 20 : 1 → 10 : 10).
(6) To the resulting crudely-purified product (309.8 mg) dissolved in anhydrous tetrahydrofuran (10 mL) was added a solution of 1.0 M tetrabutylammonium fluoride in tetrahydrofuran (1.5 mL, 1.5 mmol), and the mixture was stirred at 50°C for 3 hours under nitrogen. The reaction solution was extracted with ethyl acetate after the addition of saturated ammonium chloride aqueous solution. The organic layer was washed with saturated brine and was dried over anhydrous sodium sulfate. The resulting residue concentrated under reduced pressure was purified by thin-layer chromatography on silica gel (dichloromethane:methanol = 9:1) to afford compound No. 11 (255.2 mg). The resulting compound No. 11 (104.2 mg) was purified by reversed-phase HPLC (ODS column, mobile phase: A = 95% H₂O/CH₃CN; B = CH₃CN/MeOH = 6/4 (0.5% H₂O): B = 70%) to afford compound No. 11a (23S, 25S isomer) (8.3 mg, 10%), compound No. 11b (23R, 25R isomer) (6.6 mg, 8%), compound No. 11c (23S, 25R isomer) (3.5 mg, 3%), and compound No. 11d (23R, 25S isomer) (3.9 mg, 5 %), respectively. These four compounds are stereoisomers at the 23-position and the 25-position.
Compound No. 11a:
¹H-NMR (CDCl₃) δ : 7.33-7.20 (m, 5H), 6.39 (d, J = 11.5 Hz, 1H), 6.01 (d, J = 11.2 Hz, 1H), 5.28 (s, 1H), 5.00 (s, 1H), 4.31 (brs, 1H), 3.90-3.75 (m, 2H), 3.51-3.42 (m, 1H), 3.23-3.13 (m, 1H), 2.95-2.76 (m, 3H), 2.67 (dd, J = 13.5, 4.0 Hz, 1H), 2.32 (s, 1H), 2.28-2.19 (m, 2H), 2.05-1.80 (m, 4H), 1.74-1.11 (m, 14H), 1.08 (d, J = 6.8 Hz, 3H), 0.87 (d, J = 5.4 Hz, 3H), 0.55 (s, 3H).
Compound No. 11b:
¹H-NMR (CDCl₃) δ: 7.33-7.19 (m, 5H), 6.39 (d, J = 11.2 Hz, 1H), 6.02 (d, J = 11.2 Hz, 1H), 5.29 (s, 1H), 5.01 (s, 1H), 4.34-4.30 (m, 1H), 3.89-3.79 (m, 2H), 3.52-3.43 (m, 1H), 3.22-3.13 (m, 1H), 2.95-2.74 (m, 3H), 2.68 (dd, J = 13.5, 4.0 Hz, 1H), 2.36-2.20 (m, 3H), 2.04-1.81 (m, 5H), 1.71-0.99 (m, 13H), 1.09 (d, J = 7.1 Hz, 3H), 0.96 (d, J = 6.6 Hz, 3H), 0.55 (s, 3H).
Compound No. 11c:
¹H-NMR (CDCl₃) δ: 7.32-7.18 (m, 5H), 6.38 (d, J = 11.2 Hz, 1H), 6.01 (d, J = 11.2 Hz, 1H), 5.28 (s, 1H), 5.00 (s, 1H), 4.33-4.29 (m, 1H), 4.03-3.93 (m, 1H), 3.89-3.81 (m, 1H), 3.30-3.14 (m, 2H), 2.95-2.57 (m, 5H), 2.24 (dd, J = 13.3, 7.9 Hz, 1H), 2.14 (dd, J = 12.6, 6.2 Hz, 1H), 2.04-1.80 (m, 4H), 1.75-1.11 (m, 14H), 1.09 (d, J = 7.1 Hz, 3H), 0.89 (d, J = 6.1 Hz,3H), 0.53 (s,3H).
Compound No. 11d:
¹H-NMR (CDCl₃) δ: 7.32-7.17 (m, 5H), 6.39 (d, J = 11.2 Hz, 1H), 6.03 (d, J = 11.2 Hz, 1H), 5.29 (s, 1H), 5,02 (s, 1H), 4.34-4.29 (m, 1H), 4.03-3.94 (m, 1H), 3.89-3.80 (m, 1H), 3.34-3.15 (m, 2H), 2.95-2.58 (m, 5H), 2.28-2.16 (m, 2H), 2.06-1.87 (m, 5H), 1.80-1.00 (m, 13H), 1.09 (d, J = 6.8 Hz, 3H), 0.98 (d, J = 6.6 Hz, 3H), 0.57 (s, 3H).

### Example 2

### Production of 2α-(-hydroxypropyl)-1α,2,5-dihydroxyvitamin D₃-26,

### 23-lactam-N-(2-phenethyl) (compound No. 12)

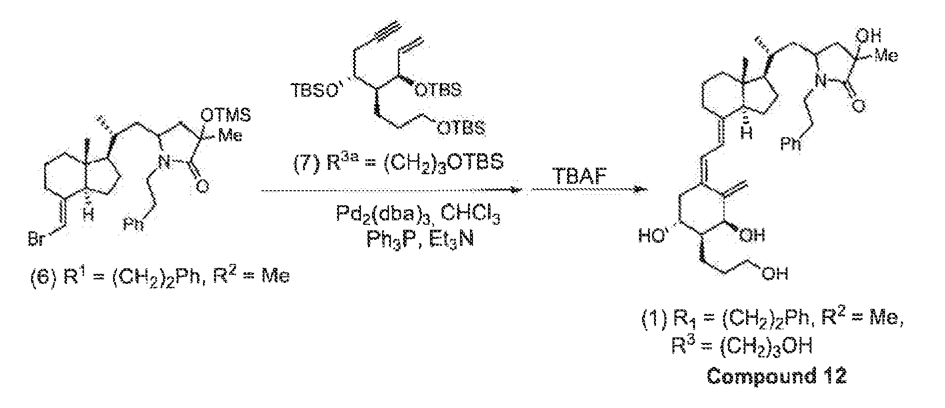

(1) Triphenylphosphine (56 mg, 0.214 mmol) and tris(dibenzylideneacetone) dipalladium (0)-chloroform adduct (28 mg, 0.027 mmol) were dissolved in a mixed solution of anhydrous toluene (4 mL) and triethylamine (4 mL), and the mixture was stirred at room temperature for 10 minutes. To the reaction solution was added the compound (6) (R¹ = (CH₂)₂Ph, R² = Me) (100 mg, 0.178 mmol) obtained in the Example 1, and a solution of compound (7) (R³=-(CH₂)₃OTBS) (144 mg, 0.267 mmol), which is synthesized according to the method of Takayama et al. (Tetrahedron, 60: 7951-7961, 2004) obtainable in literature (e.g., J. Org. Chem. 66: 8760-8771, (2001)), in anhydrous toluene (4 mL) solution, and the mixture was stirred at 120°C for 2 hours under argon. The reaction solution was extracted with ethyl acetate after the addition of saturated ammonium chloride aqueous solution. The aqueous layer was adjusted to weak basic by the addition of saturated sodium bicarbonate aqueous solution and the resulting solution was extracted with ethyl acetate. The combined organic layer was washed with saturated brine and dried over anhydrous sodium sulfate. The resulting residue concentrated under reduced pressure was purified crudely by thin-layer chromatography on silica gel (n-hexane:ethyl acetate = 4 : 1).
(2) To the resulting crudely purified product (128.4 mg) dissolved in anhydrous tetrahydrofuran (6 mL) was added a solution of 1.0 M tetrabutylammonium fluoride in tetrahydrofuran (0.5 mL, 0.5 mmol), and the mixture was stirred at 50°C for 1 hour under nitrogen. The reaction solution was extracted with ethyl acetate after the addition of a saturated ammonium chloride aqueous solution. The organic layer was washed with saturated brine and was dried over anhydrous sodium sulfate. The resulting residue concentrated under reduced pressure was purified by thin-layer chromatography on silica gel (dichlorometbane:methanol = 5:1) to afford compound No. 12 (126.3 mg). The resulting compound No. 12 (63.2 mg) was purified by reversed-phase HPLC (ODS column, mobile phase; A = 95% H₂O/CH₃CN; B = CH₃CN/MeOH = 6/4 (0.5% H₂O); B = 75%) to afford compound No. 12a (23S, 25S isomer) (4.0 mg, 7%), compound No. 12b (23R, 25R isomer) (2.2 mg, 4%), compound No. 12c (23S, 25R isomer) (2.0 mg, 4%), and compound No. 12d (23R, 25S isomer) (2.2 mg, 6%), respectively. These four compounds are stereoisomers at the 23-position and the 25-position.
Compound No. 12a:
¹H-NMR (CDCl₃) δ: 7.33-7.20 (m, 5H), 6.40 (d, J = 11.2 Hz, 1H), 6.00 (d, J = 11.5 Hz, 1H), 5.27 (s, 1H), 4.99 (s, 1H), 4.39 (brs, 1H), 3.94-3.86 (m, 1H), 3.83-3.67 (m, 3H), 3.51-3.42 (m, 1H), 3.23-3.13 (m, 1H), 2.94-2.76 (m, 3H), 2.67 (dd, J = 13.7 Hz, 4.1 Hz, 1H), 2.30-2.19 (m, 2H), 2.03-1.95 (m, 2H), 1.90-1.10 (m, 22H), 0.87 (d, J = 5.6 Hz, 3H), 0.54 (s, 3H).
Compound No. 12b:
¹H-NMR (CDCl₃) δ: 7.33-7.19 (m, 5H), 6.40 (d, J = 11.5 Hz, 1H), 6.01 (d, J = 11.2 Hz, 1H), 5.29 (s, 1H), 5.00 (s, 1H), 4.39 (brs, 1H), 3.95-3.78 (m, 2H), 3.74-.3.67 (m, 2H), 3.52-3.42 (m, 1H), 3.22-3.12 (m, 1H), 2.95-2.74 (m, 3H), 2.67 (dd, J = 13.3, 4.0 Hz, 1H), 2.36-2.22 (m, 2H), 2.03-194 (m, 2H), 1.88-0.98 (m, 22H), 0.96 (d, J = 6.6 Hz, 3H), 0.55 (s, 3H).
Compound No. 12c:
¹H-NMR (CDCl₃) δ: 7.32-7.17 (m, 5H), 6.39 (d, J = 11.0 Hz, 1H), 5.99 (d, J = 11.2 Hz, 1H), 5.28 (s, 1H), 4.99 (s, 1H), 4.38 (s, 1H), 4.04-3.85 (m, 2H), 3.75-3.67 (m, 2H), 3.35-3.14 (m, 2H), 2.96-2.60 (m, 4H), 2.30-2.21 (m, 1H), 2.14 (dd, J = 12.3 Hz, 6.2 Hz, 1H), 2.04-1.95 (m, 2H), 1.90-1.10 (m, 22H), 0.89 (d, J = 6.1 Hz, 3H), 0.53 (s, 3H).
Compound No. 12d:
¹H-NMR (CDCl₃) δ: 7.32-7.17 (m, 5H), 6.40 (d, J = 11.2 Hz, 1H), 6.01 (d, J = 11.5 Hz, 1H), 5.29 (s, 1H), 5.00 (s, 1H), 4.39 (s, 1H), 4.03-3.85 (m, 2H), 3.74-3.68 (m, 2H), 3.53-3.15 (m, 2H), 2.95-2.80 (m, 2H), 2.78-2.60 (m, 3H), 2.30-2.15 (m, 2H), 2.05-1.87 (m, 4H) 1.80-1.00 (m, 19H), 0.98 (d, J = 6.6 Hz, 3H), 0.56 (s, 3H).

### Example 3

### Production of 2α-(3-hydroxypropoxy)-1α,25-dihydroxyvitamin D₃-26,

### 23-lactam-N-(2-phenethyl) (compound No. 13)

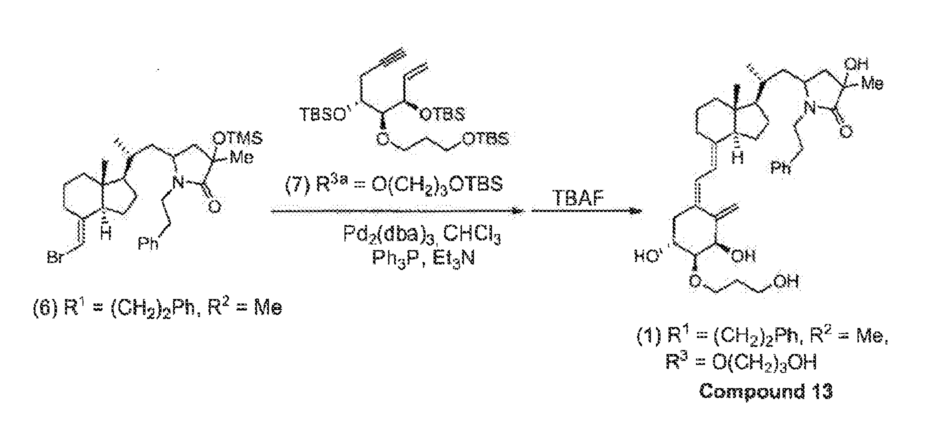

(1) Triphenylphosphine (112 mg, 0.428 mmol) and tris(dibenzylideneacetone) dipalladium (0)-chloroform adduct (56 mg, 0.054 mmol) were dissolved in a mixed solution of anhydrous toluene (4 mL) and triethylamine (4 mL) and the mixture was stirred at room temperature for 10 minutes. To the reaction mixture was added the compound (6) (R¹ = (CH₂)₂Ph, R² = Me) (200 mg, 0.357 mmol) obtained in Example 1 and a solution of the compound (7) (R³=O(CH₂)₃OTBS) (240 mg, 0.428 mmol), which is synthesized according to the method of Takayama et al. (Tetrahedron, 60: 7951-7961, 2004) obtainable in literature (e.g., Org. Lett. 2: 2619-2622, (2000)), in anhydrous toluene (4 mL) and the mixture was stirred at 120°C for 3 hours under argon. Saturated ammonium chloride aqueous solution was added to the reaction solution and the mixture was extracted with ethyl acetate. Saturated sodium bicarbonate aqueous solution was added to the aqueous layer to adjust to weak basic, and the mixture then was extracted with ethyl acetate. The combined organic layer was washed with saturated brine and was dried over anhydrous sodium sulfate. The resulting residue concentrated under reduced pressure was purified crudely by column chromatography on silica gel (n-hexane:ethyl acetate =20:1 → 10:1).
(2) The resulting crudely purified product (251.2 mg) was dissolved in anhydrous tetrahydrofuran (10 mL), a solution of 1.0 M tetrabutylammonium fluoride in tetrahydrofuran (1.0 mL, 1.0 mmol) was added to the mixture, and the resulting mixture was stirred at 50°C for 2 hours under nitrogen. The reaction solution was extracted with ethyl acetate after the addition of a saturated ammonium chloride aqueous solution. The organic layer was washed with saturated brine and was dried over anhydrous sodium sulfate. The resulting residue concentrated under reduced pressure was purified by thin-layer chromatography on silica gel (dichloromethane:methanol = 5:1) to afford compound No. 13 (215.4 mg). The resulting compound No. 13 (53.9 mg) was purified by reversed-phase HPLC (ODS column, mobile phase: A = 95% H₂O/CH₃CN; B = CH₃CN/MeOH = 6/4 (0.5% H₂O); B = 75%) to afford compound No. 13a (23S, 25S isomer) (6.7 mg, 11%), compound No. 13b (23R, 25R isomer) (3.2 mg, 6%), compound No. 13c (23S, 25R isomer) (2.5 mg, 5%), and compound No. 13d (23R, 25S isomer) (3.0 mg, 5%), respectively. These four compounds are stereoisomers at the 23-position and the 25-position.
Compound No. 13a:
¹H-NMR (CDCl₃) δ: 7.33-7.19 (m, 5H), 6.41 (d, J = 11.2 Hz, 1H), 6.02 (d, J = 11.2 Hz, 1H), 5.39 (s, 1H), 5.08 (s, 1H), 4.45 (s, 1H), 4.10-4.02 (m, 1H), 3.93-3.69 (m, 6H), 3.52-3.42 (m, 1H), 3.38 (dd, J = 7.4 Hz, 3.3 Hz, 1H), 3.23-3,14 (m, 1H), 2.93-2.75 (m, 2H), 2.73-2.64 (m, 2H), 2.55 (brs, 2H), 2.28-2.16 (m, 2H), 2.03-1.94 (m, 2H), 1.92-1.81 (m, 2H), 1.70-1.10 (m, 14H), 0.86 (d, J = 5.6 Hz, 3H), 0.54 (s, 3H).
Compound No. 13b:
¹H-NMR (CDCl₃) δ: 7.33-7.18 (m, 5H), 6.42 (d, J = 11.2 Hz, 1H), 6.02 (d, J = 11.5 Hz, 1H), 5.40 (s, 1H), 5.10 (s, 1H), 4.46 (s, 1H), 4.10-4.03 (m, 1H), 3.93-3.75 (m, 6H), 3.50-3.42 (m, 1H), 3.39 (dd, J = 7.4 Hz, 3.3 Hz, 1H), 3.23-3.12 (m, 1H), 2.95-2.74 (m, 4H), 2.69 (dd, J = 13.9, 4.4 Hz, 1H), 2.60-2.15 (m, 3H), 2.02-0.97 (m, 18H), 0.96 (d, J = 6.9 Hz, 3H), 0.55 (s, 3H).
Compound No. 13c:
¹H-NMR (CDCl₃) δ: 7.32-7.18 (m, 5H), 6.41 (d, J = 11.2 Hz, 1H), 6.01 (d, J = 11.5 Hz, 1H), 5.39 (s, 1H), 5.09 (s, 1H), 4,45 (s, 1H), 4.10-3.73 (m, 6H), 3.37 (dd, J = 7.4, 3.3 Hz, 1H), 3.30-3.14 (m, 2H), 2.94-2.40 (m, 7H), 2.29-2.10 (m, 3H), 2.03-1.79 (m, 6H), 1.70-1.10 (m, 14H), 0.88 (d, J = 6.3 Hz, 3H), 0.53 (s, 3H).
Compound No. 13d:
¹H-NMR (CDCl₃) δ: 7.32-7.17 (m, 5H), 6.42 (d, J = 11.2 Hz, 1H), 6.03 (d, J = 11.2 Hz, 1H), 5.41 (s, 1H), 5.10 (s, 1H), 4.46 (s, 1H), 4.10-3.74 (m, 7H), 3.39 (dd, J = 7.4, 3.3 Hz, 1H), 3.32-.3.15 (m, 2H), 2.95-2.43 (m, 6H), 2.28-2.16 (m, 2H), 2.03-1.00 (m, 18H), 0.97 (d, J = 6.3 Hz, 3H), 0.56 (s, 3H).

### Example 4

### Production of 2α-methyl-1α,25-dihydroxyvitamin D₃-26,

### 23-lactam-N-(3,5-diethoxybenzyl) (compound No. 41)

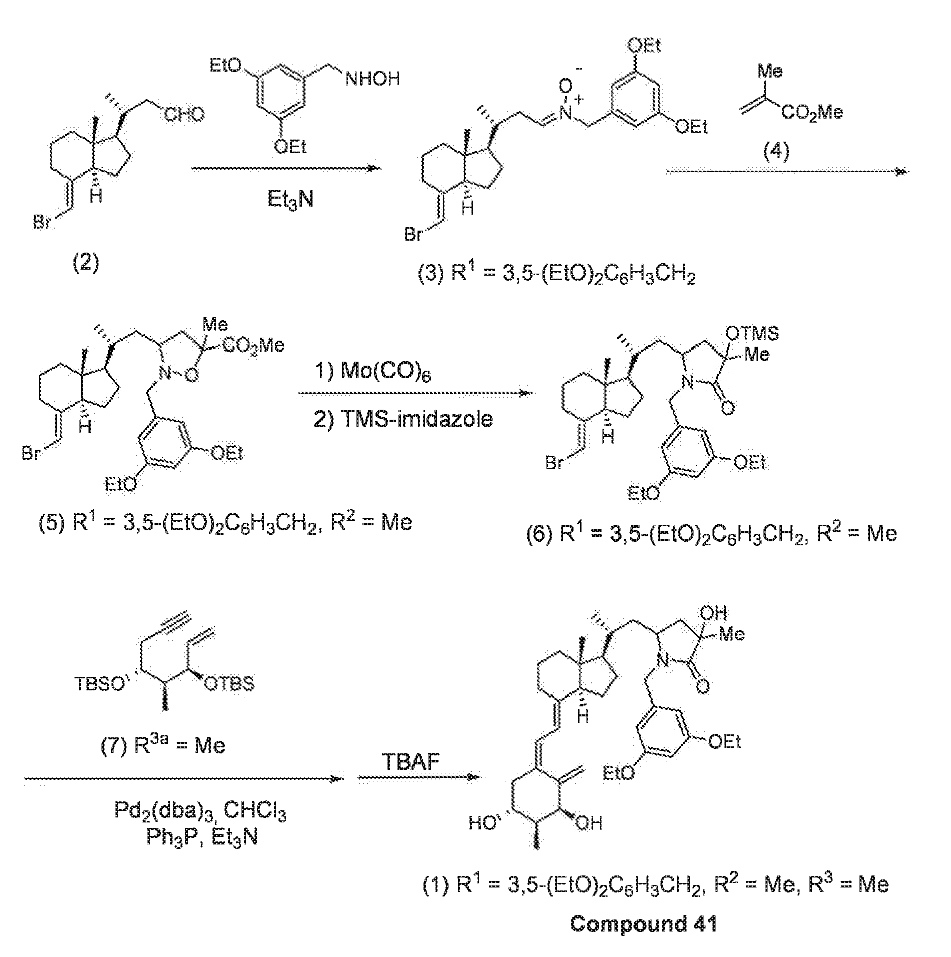

(1) To a solution of the compound (2) (200 mg, 0.67 mmol) which is obtained by the method described in literature (e.g., the description of the International Publication WO95/33716) in dichloromethane (1.3 mL) was added 3,5-diethoxyhydroxylamine (169.4 mg, 0.80 mmol) which is obtained in the Reference Example 2 and Et₃N (230 µL, 1.65 mmol), and the mixture was stirred at room temperature for 2 hours under nitrogen. The reaction solution was extracted with ethyl acetate after adding a saturated ammonium chloride aqueous solution. The organic layer was dried over anhydrous sodium sulfate and the solvent was evaporated after filtration. The resulting residue was purified by flash column chromatography (n-hexane:ethyl acetate = 1:4) to afford compound (3) (R¹ = 3,5-(EtO)₂C₆H₃CH₂) (312.3 mg, 95%).
(2) To a solution of the compound (3) (R¹ = 3,5-(EtO)₂C₆H₃CH₂) (294.6 mg) in toluene (9 mL) was added methyl methacrylate (4) (R² = R = Me) (3.640 µL, 5.98 mmol) and the mixture was stirred at 90°C for 1.5 hours under nitrogen. After the mixture was cooled to room temperature, the solution was evaporated and the residue was purified by flash column chromatography (n-hexane:ethyl acetate = 10: 1 → 6:1) to afford compound (5) (R¹ = 3,5-(EtO)₂C₆H₃CH₂, R² = R = Me) (321.9 mg, 91 %).
   ¹H-NMR (CDCl₃) δ: 6.60-6.49 (m, 2H), 6.33 (s, 1H), 5.63 (s, 1H), 4.01-3.95 (m, 4H), 3.77-3.75 (m, 3H), 3.64-3.61 (m, 1H), 3.17-3.09 (m, 1H), 2.93-2.84 (m, 1H), 1.61-1.47 (m, 3H), 1.42-1.35 (m, 6H), 2.64-1.11 (m, 17H), 0.95-0.65 (m, 3H), 0.56-0.51 (m, 3H).
(3) The resulting compound (5) (R¹ = 3,5-(EtO)₂C₆H₃CH₂, R² = R = Me) (172.5 mg, 0.29 mmol) was dissolved in a mixed solution of acetonitrile:water = 7 : 1 (3 mL), molybdenum hexacarbonyl (230.6 mg, 0.87 mmol) was added and the mixture was stirred at 90°C for 3.5 hours. After cooling the reaction mixture to room temperature, the reaction mixture was filtered on Celite and concentrated under reduced pressure. The resulting residue was purified by column chromatography on silica gel (n-hexane:ethyl acetate = 2:1) to afford a lactam reduction product (111.5 mg, 68%).
   ¹H-NMR (CDCl₃) δ: 6.26-6.17 (m, 3H), 5.54 (m, 1H), 4.88-4.76 (m, 1H), 3.87 (q, J = 7.2 Hz, 4H), 3.79-3.72 (m, 1H), 3.53-3.16 (m, 1H), 2.79-2.74 (m, 1H), 1.41-1.39 (m, 3H), 1.31-1.25 (m, 6H), 2.26-0.94 (m, 17H), 0.81-0.69 (m, 3H), 0.45-0.40 (m, 3H).
(4) The resulting lactam reduction product (171.6 mg, 0.31 mmol) was dissolved in dichloromethane (10 mL), to which was added trimethylsilylimidazole (224 µL, 1.53 mmol), and the resulting mixture was stirred at room temperature for 1 hour under nitrogen. Water was added to the reaction solution at 0°C under stirring, and the mixture was extracted with dichloromethane. The organic layer was dried over anhydrous magnesium sulfate. After filtration, the resulting residue concentrated under reduced pressure was purified by column chromatography on silica gel (n-hexane:ethyl acetate = 10:1) to afford compound (6) (R¹ = 3,5-(EtO)₂C₆H₃CH₂, R² = Me) (185.9 mg, 96%) as an oily product.
   ¹H-NMR (CDCl₃) δ: 6.35-6.28 (m, 3H), 5.63 (m, 1H), 4.95-4.80 (m, 1H), 3.98-3.94 (m, 4H), 3.89-3.79 (m, 1H), 3.59-3.18 (m, 1H), 2.91-2.82 (m, 1H), 1.48 (s, 3H), 1.39-1.36 (m, 6H), 2.34-1.09 (m, 17H), 0.92-0.79 (m, 3H), 0.55-0.49 (m, 3H), 0.19-0.15 (m, 9H).
(5) The resulting compound (6) (R¹ = 3,5-(EtO)₂C₆H₃CH₂, R² = Me) (28.3 mg, 0.045 mmol) and the compound (7) (R^{3a} = Me) (16.3 mg, 0.0426 mmol) which is obtained by the method described in literature (e.g., J. Med. Chem. 43: 4247-4265 (2000)) were dissolved in a mixed solvent of anhydrous toluene:triethylamine =1:1 (2 mL). To the mixture was added tetrakis(triphenylphosphine)palladium and the resulting mixture was stirred at 110°C for 2 hours under argon. The reaction solution was cooled to room temperature and concentrated under reduced pressure. The resulting residue was purified by column chromatography on silica gel (n-hexane:ethyl acetate = 10:1) to afford a compound (22.6 mg, 54 %).
(6) To the resulting compound (22.6 mg, 0.024 mmol) dissolved in anhydrous tetrahydrofuran (1.5 mL) was added triethylaminetris(hydrofluoride) (1.6 mL, 0.0098 mmol) at room temperature, and the mixture was stirred for 50 hours while increasing the temperature to 40°C. The reaction solution was extracted with ethyl acetate after the addition of a saturated sodium bicarbonate aqueous solution at 0°C and stirring. The organic layer was washed with saturated brine and was dried over anhydrous magnesium sulfate. The resulting residue concentrated under reduced pressure was purified by column chromatography on silica gel (n-hexane:ethyl acetate = 1:2 → 1:3) to afford compound No. 41 (12.4 mg, 81%). The compound No. 41 (20 mg) obtained in a similar manner was purified by reversed phase HPLC (ODS column, mobile phase: A = 95% H₂O/CH₃CN ; B = CH₃CN/MeOH = 6/4 (0.5% H₂O); B = 60%) to afford compound No. 41a (2.6 mg), compound No. 41b (3.1 mg), a compound No. 41c (3.2 mg), and compound No. 41d (3.7 mg), respectively. These four compounds are stereoisomers at the 23-position and the 25-position.
Compound No. 41a:
¹H-NMR (CDCl₃) δ: 6.38 (d, J = 11.5 Hz, 1H), 6.34 (t, J = 2.1 Hz, 1H), 6.273 (s, 1H), 6.267 (s, 1H), 6.00 (d, J =11.2 Hz, 1H), 5.28 (s, 1H), 5.00 (s, 1H), 4.92 (d, J = 15.5 Hz, 1H), 4.32 (t, J = 4.1 Hz, 1H), 3.92-4.00 (m, 5H), 3.81-3.89 (m, 1H), 3.27-3.35 (m, 1H), 2.78-2.84 (m, 1H), 2.64-2.71 (m, 2H), 2.20-2.30 (m, 2H), 1.38 (t, J = 7.0 Hz, 6H), 1.25-2.05 (m, 18H), 1.08 (d, J = 7.0 Hz, 3H), 0.89 (d, J = 7.5 Hz, 3H), 0.49 (s, 3H).
Compound No. 41b:
¹H-NMR (CDCl₃) δ: 6.38 (d, J = 11.5 Hz, 1H), 6.31-6.35 (m, 3H), 6.00 (d, J = 11.2 Hz, 1H), 5.29 (s, 1H), 5.00 (s, 1H), 4.90 (d, J = 15.5 Hz, 1H), 4.32 (t, J = 4.0 Hz, 1H), 3.98 (m, 1H), 3.97 (q, J = 7.0 Hz, 4H), 3.82-3.89 (m, 1H), 3.47-3.56 (m, 1H), 2.78-2.85 (m, 1H), 2.67 (dd, J = 13.0 and 4.0Hz, 1H), 2.50 (m, 1H), 2.36 (dd, J = 13.0 and 7.5 Hz, 1H), 2.23 (dd, J = 13.0 and 7.5 Hz, 1H), 1.86-1.97 (m, 5H), 1.38 (t, J = 7.0 Hz, 6H), 1.08 (d, J = 7.0 Hz, 3H), 0.90-1.85 (m, 13 H), 0.89 (d, J = 7.5 Hz, 3H), 0.49 (s, 3H).
Compound No. 41c:
¹H-NMR (CDCl₃) δ: 6.38 (d, J = 11.5 Hz, 1H), 6.35 (m, 3H), 6.01 (d, J = 11.2 Hz, 1H), 5.28 (s, 1H), 5.00 (s, 1H), 4.91 (d, J = 15.5 Hz, 1H), 4.31 (t, J = 4.0 Hz, 1H), 3.98 (m, 1H), 3.97 (q, J = 7.0 Hz, 4H), 3.82-3.89 (m, 1H), 3.49-3.57 (m, 1H), 2.78-2.85 (m, 1H), 2.67 (dd, J = 13.0 and 4.0 Hz, 1H), 2.47 (m, 1H), 2.20-2.30 (m, 2H), 1.80-2.02 (m, 5H), 1.38 (t, J = 7.0 Hz, 6H), 1.08 (d, J = 7.0 Hz, 3H), 1.10-1.75 (m, 13H) 0.81 (d, J = 7.5 Hz, 3H), 0.53 (s, 3H).
Compound No. 41d:
¹H-NMR (CDCl₃) δ: 6.38 (d, J = 11.5 Hz, 1H), 6.35 (t, J = 2.1 Hz, 1H), 6.303 (s, 1H), 6.297 (s, 1H), 6.00 (d, J = 11.2 Hz, 1H), 5.28 (s, 1H), 5.00 (s, 1H), 4.93 (d, J = 15.5 Hz, 1H), 4.31 (t, J = 4.0 Hz, 1H), 3.98 (m, 1H), 3.97 (q, J = 7.0 Hz, 4H), 3.82-3.89 (m, 1H), 3.27-3.37 (m, 1H), 2.78-2.86 (m, 1H), 2.73 (s, 1H), 2.67 (dd, J = 13.0 and 4.0 Hz, 1H), 2.17-2.27 (m, 2H), 1.39 (t, J = 7.0 Hz, 6H), 1.08 (d, J = 7.0 Hz, 3H), 1.10-2.00 (m, 13H), 0.80 (d, J = 7.5 Hz, 3H), 0.51 (s, 3H).

### Example 5

### Production of 2α-(3-hydroxypropyl)-1α,25-dihydroxyvitamin

### D₃-26,23-lactam-N-(3,5-diethoxybenzyl) (compound No. 42)

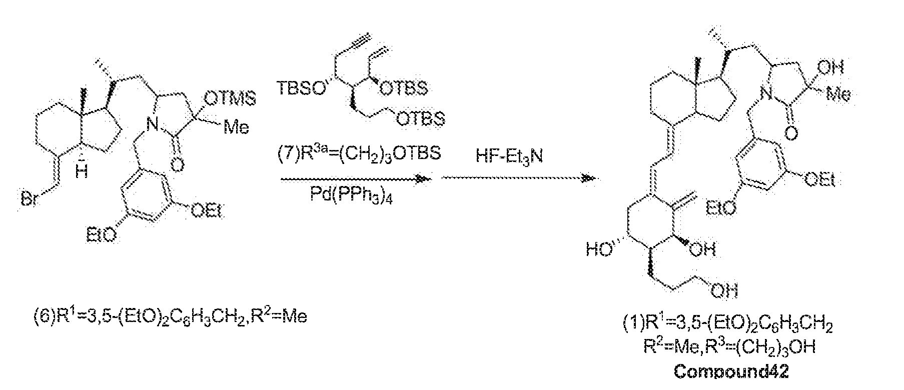

(1) The compound (6) (R¹ = 3,5-(ETO)₂ C₆H₃CH₂, R² = Me) (28.1 mg, 0.0443 mmol) obtained in the Example 4 and the compound (7) (R³ = (-CH₂)₃OTBS) (21.8 mg, 0.0403 mmol), which is synthesized by the method of Takayama et al. (Tetrahedron, 60: 7951-7961, 2004) obtainable by the method described in literature (e.g., J. Org. Chem. 66: 8760-8771 (2001)), were dissolved in a mixed solution of anhydrous toluene:triethylamine = 1:1 (2.2 mL). The mixture was added to tetrakis(triphenylphosphine)palladium and the resulting mixture was stirred at 90°C for 4 hours under argon. The reaction solution was cooled to room temperature and concentrated under reduced pressure. The resulting residue was purified by column chromatography on silica gel (n-hexane:ethyl acetate = 100:0 → 10:1 → 8:1) to obtain a crude product (40.7 mg, 88%).
(2) To a solution of the crude product (40.7 mg, 0.0372 mmol) dissolved in tetrahydrofuran (2.4 mL) was added triethylaminetris(hydrofluoride) (1.3 mL, 0.0082 mmol) at room temperature and the mixture was stirred for 43 hours by increasing the temperature to 40°C. The mixture was extracted with ethyl acetate after the addition of a saturated sodium bicarbonate aqueous solution at 0°C under stirring. The organic layer was washed with saturated brine and dried over anhydrous magnesium sulfate. The resulting residue concentrated under reduced pressure was purified by column chromatography on silica gel (n-hexane:ethyl acetate = 1:8 → ethyl acetate → chloroform:methanol = 9:1 → 5:1) to afford compound No. 42 (21.37 mg, 84 %). The resulting compound No. 42 (21.37 mg) was purified by normal-phase HPLC (PEGASIL 60-5, mobile phase:hexane:ethyl acetate:2-propanol = 10:85:5) to afford compound No. 42a (4.16 mg), compound No. 42b (1.33 mg), compound No. 42c (3.0mg), respectively. These three compounds are stereoisomers at the 23-position and the 25-position.
Compound No. 42a:
¹H-NMR (CDCl₃) δ: 6.33 (d, J = 11.5 Hz, 1H), 6.31 (t, J = 2.1 Hz, 1H), 6.31 (s, 2H), 6.00 (d, J = 11.2 Hz, 1H), 5.34 (s, 1H), 5.00 (d, J = 2.1 Hz, 1H), 4.96 (d, J = 15.5 Hz, 1H), 4.40 (brs, 1H), 3.96 (q, J = 6.9 Hz, 4H), 3.70 (m, 2H), 3.53 (m, 1H), 3.10 (q, J = 7.3 Hz, 1H), 2.83 (m, 1H), 2.65 (m, 1H), 2.60 (m, 1H), 2.50-2.20 (m, 3H), 2.20-1.20 (m, 19H), 1.38 (t, J = 7.0 Hz, 6H), 1.11-1.10 (m, 3H), 0.89 (d, J = 7.0 Hz, 3H), 0.48 (s, 3H).
Compound No. 42b:
¹H-NMR (CDCl₃) δ: 6.39 (d, J = 11.5 Hz, 1H), 6.34 (s, 1H), 6.26 (s, 2H), 6.00 (d, J = 11.2 Hz, 1H), 5.34 (m, 1H), 5.33 (s, 1H), 5.00 (s, 1H), 4.96 (d, J = 15.5 Hz, 1H), 4.50 (brs, 1H), 3.96 (q, J = 5.9 Hz, 4H), 3.70 (m, 2H), 3.53 (m, 1H), 3.10 (q, J = 6.0 Hz, 1H), 2.83 (m, 1H), 2.65 (m, 1H), 2.50-2.20 (m, 4H), 2.10-1.90 (m, 3H), 1.80-1.20 (m, 18H), 1.38 (t, J = 7.0 Hz, 6H), 0.89 (d, J = 7.0 Hz, 3H), 0.47 (s, 3H).
Compound No. 42c:
¹H-NMR (CDCl₃) δ: 6.39 (d, J = 11.5 Hz, 1H), 6.37 (s, 3H), 6.00 (d, J = 11.2 Hz, 1H), 5.34 (m, 1H), 5.33 (s, 1H), 5.00 (s, 1H), 4.96 (d, J = 15.5 Hz, 1H), 4.50 (brs, 1H), 3.96 (q, J = 5.9 Hz, 4H), 3.70 (m, 2H), 3.53 (m, 1H), 3.10 (q, J = 6.0 Hz, 1H), 2.83 (m, 1H), 2.65 (m, 1H), 2.60 (m, 1H), 2.50-2.20 (m, 3H), 2.10-1.90 (m, 3H), 1.80-1.20 (m, 16H), 1.38 (t, J = 7.0 Hz, 6H), 1.11-1.10 (m, 3H), 0.89 (d, J = 7.0 Hz, 3H), 0.52 (s, 3H).

### Example 6

### Production of 2α-(3-hydroxypropoxy)-1α,25-dihydroxyvitamin D₃-26,

### 23-lactam-N-(3,5-diethoxybenzyl) (compound No. 43)

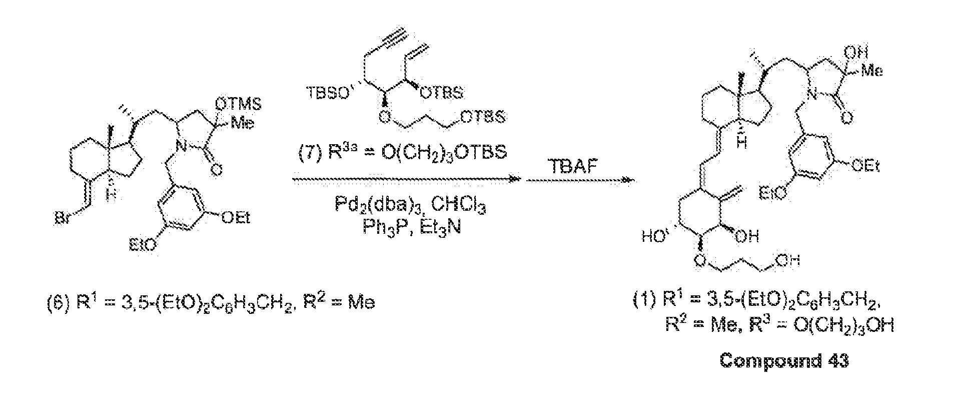

(1) The compound (6) (R¹ = 3,5-(EtO)₂C₆H₃CH₂, R² =Me) (29.8 mg, 0.047 mmol) obtained in Example 4 and the compound (7) (R³ = -O(CH₂)₃OTBS) (23.8 mg, 0.043 mmol) which is obtained by the method described in literature (e.g., J. Med. Chem., 43:4247-4265, 2000) were dissolved in a mixed solvent of anhydrous toluene:triethylamine =1:1 (2 mL). The mixture was added to tetrakis(triphenylphosphine)palladium under argon and the resulting mixture was stirred at 90°C for 2 hours. After cooling the reaction mixture to room temperature, the mixture was concentrated under reduced pressure. The resulting residue was purified by column chromatography on silica gel (n-hexane:ethyl acetate = 20:1 → 10:1) to obtain a product (28.8 mg, 55%).
(2) To the resulting product (24.1 mg, 0.022 mmol) dissolved in tetrahydrofuran (1.5 mL), triethylaminetris(hydrofluoride) (1.2 mL, 0.0074 mmol) was added at room temperature and the mixture was stirred for 48 hours by increasing the temperature to 40°C. To the reaction solution was added a saturated sodium bicarbonate aqueous solution at 0°C under stirring, and the resulting mixture was extracted with ethyl acetate. The organic layer was washed with saturated brine and dried over anhydrous magnesium sulfate. The resulting residue concentrated under reduced pressure was purified by column chromatography on silica gel (n-hexane:ethyl acetate = 1:6 → 1:8) to afford compound No. 43 (11.8 mg, 78%). The compound No. 43 (20 mg) obtained in a similar manner was purified by reversed-phase HPLC (ODS column, mobile phase: A = 95% H₂O/CH₃CN; B = CH₃CN/MeOH = 6/4 (0.5% H₂O); B = 60%) to afford compound No. 43a (2.8 mg), compound No. 43b (2.9 mg), compound No. 43c (2.0 mg), and compound No. 43d (2.9 mg), respectively. These four compounds are stereoisomers at the 23-position and the 25-position.
Compound No. 43a:
¹H-NMR (CDCl₃) δ: 6.41 (d, J = 11.5 Hz, 1H), 6.34 (t, J = 2.1 Hz, 1H), 6.270 (s, 1H), 6,265 (s, 1H), 6.00 (d, J = 11.2 Hz, 1H), 5.39 (s, 1H), 5.09 (d, J = 2.1 Hz, 1H), 4.92 (d, J = 15.5 Hz, 1H), 4.45 (t, J = 4.1 Hz, 1H), 4.02-4.10 (m, 1H), 3.92.-4.00 (m, 5H), 3.75-3.92 (m, 6H), 3.40 (dd, J = 3.5 and 7.0 Hz,1H), 3.23-3.35 (m, 1H), 2.77-2.84 (m, 1H), 2.75 (s, 1H), 2.69 (dd, J = 4.5 and 14.0 Hz, 1H), 2.57 (d, J = 4.5 Hz, 1H), 2.44 (s, 1H), 2.10.-2.30 (m, 3H), 1.38 (t, J = 7.0 Hz, 6H), 1.20-2.00 (m, 11H), 1.01-1.11 (m, 3H), 0.89 (d, J = 7.0 Hz, 3H), 0.48 (s, 3H).
Compound No. 43b:
¹H-NMR (CDCl₃) δ: 6.41 (d, J = 11.5 Hz, 1H), 6.31-6.35(m, 3H), 6.00 (d, J = 11.2 Hz, 1H), 5.40 (s, 1H), 5.09 (d, J = 2.1 Hz, 1H), 4.89 (d, J = 15.5 Hz, 1H), 4.45 (t, J = 4.1 Hz, 1H), 4.03-4.10 (m, 1H), 3.97 (q, J = 7.0 Hz, 4H), 3.97 (m, 1H), 3.75-3.92 (m, 6H), 3.48-3.55 (m, 1H), 3.39 (dd, J = 3.5 and 7.0 Hz, 1H), 2.78-2.84 (m,1H), 2.69 (dd, J = 4.5 and 14.0 Hz, 1H), 2.58 (d, J = 4.5 Hz, 1H), 2.52 (s, 1H), 2.44 (s, 1H), 2.36 (dd, J = 8.0 and 13.5 Hz, 1H), 2.19-2.28 (m, 2H), 1.84-1.98 (m, 5H), 1.73-1.81 (m, 2H), 1.35-1.70 (m, 2.H), 1.38 (t, J = 7.0 Hz, 6H), 0.96-1.30 (m, 5H), 0.88 (d, J = 7.0 Hz, 3H), 0.49 (s, 3H).
Compound No. 43c:
¹H-NMR (CDCl₃) δ : 6.41 (d, J = 11.5 Hz, 1H), 6.35 (s, 3H), 6.01 (d, J = 11.2 Hz, 1H), 5.39 (s, 1H), 5.09 (d, J = 2.1 Hz, 1H), 4.91 (d, J = 15.5 Hz, 1H), 4.45 (t, J = 4.1 Hz, 1H), 4.02-4.10 (m, 1H), 3.97 (q, J = 7.0 Hz, 1H), 3.97 (m, 1H), 3.75-3.92 (m, 6H), 3.49-3.58 (m, 1H), 3.39 (dd, J = 3.5 and 7.0 Hz, 1H), 2.78-2.85 (m, 1H), 2.68 (dd, J = 4.5 and 14. 0 Hz, 1H), 2.55 (d, J = 4.5 Hz, 1H), 2.43 (s, 2H), 2.12-2.30 (m, 3H), 1.92-2.01 (m, 3H), 1.79-1.92 (m, 3H), 1.38 (t, J = 7.0 Hz, 6H), 1.10-1.70 (m, 8H), 0.80 (d, J = 7.0 Hz, 3H), 0.53 (s, 3H).
Compound No. 43d:
¹H-NMR (CDCl₃) δ: 6.41 (d, J = 11.5 Hz, 1H), 6.35 (t, J = 2.1 Hz, 1H), 6.302 (s, 1H), 6.297 (s, 1H), 6.01 (d, J = 11.2 Hz, 1H), 5.39 (s, 1H), 5.08 (d, J = 2.1 Hz, 1H), 4.93 (d, J = 15.5 Hz, 1H), 4.45 (br, 1H), 4.02-4.10 (m, 1H), 3.92-4.01 (m, 5H), 3.74-3.92 (m, 6H) 3.37 (dd, J = 3.5 and 7.0 Hz, 1H), 3.28-3.37 (m, 1H), 2.78-2.86 (m, 1H), 2.74 (m, 1H), 2.68 (dd, J = 4.5 and 14.0 Hz, 1H), 2.55 (d, J = 4.5 Hz, 1H), 2.47 (s, 1H), 2.18-2.28 (m, 3H), 1.39 (t, J = 7.0 Hz, 6H), 1.10-2.00 (m, 14H), 0.79 (d, J = 7.0 Hz, 3H), 0.51 (s, 3H).

### Example 7

### Production of 2α-methyl-1α,25-dihydroxyvitamin D₃-26,

### 23-lactam-N-(2-naphthylethyl) (compound No. 61)

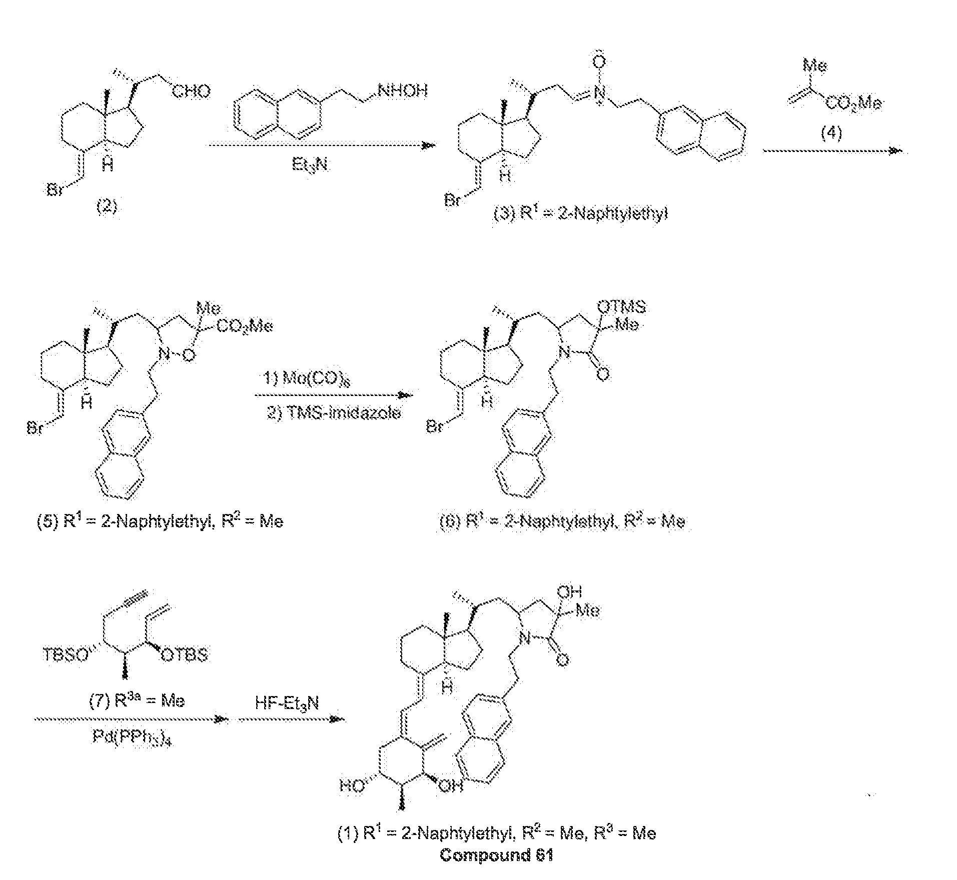

(1) To a solution of the compound (2) (570 mg, 1.91 mmol) which is obtained according to the method described in literature (e.g., the description of the International Publication WO95/33716) in dichloromethane (3.8 mL) were added 2-naphthylethylamine (464 mg, 2.48 mmol) obtained in the Reference Example 3 and Et₃N (1063 µL, 7.62 mmol) and the mixture was stirred at room temperature for 17.5 hours under nitrogen. A saturated ammonium chloride aqueous solution was added to the mixture and the resulting mixture was extracted with ethyl acetate. The organic layer was dried over anhydrous sodium sulfate and the solvent was evaporated after filtration. The resulting residue was purified by column chromatography on silica gel (n-hexane:ethyl acetate = 1:4 → 1:8 → ethyl acetate → chloroform:methanol = 9: 1) to afford compound (3) (706 mg, 79%).
(2) To a solution of the compound (3) (R¹ = 2-naphthylethyl) (706 mg) in toluene (21 mL) was added methyl methacrylate (4) (R² = R = Me) (1.6 mL, 15.07 mmol) and the resulting mixture was stirred at 90°C for 12 hours under nitrogen. After cooling the mixture to room temperature, the solvent was evaporated and the residue was purified by column chromatography on silica gel (n-hexane:ethyl acetate = 30:1 → 6:1) to afford compound (5) (R¹ = 2-naphthylethyl, R² = R= Me) (775.6 mg, 91%).
(3) To the solution of the above compound (5) (750.5 mg, 1.32 mmol) dissolved in mixed solvent of acetonitrile:water = 7:1 (13mL) was added molybdenum hexacarbonyl (2090.8 mg, 7.92 mmol) and the mixture was stirred at 90°C for 7 hours. After cooling the reaction solution to room temperature, the solution was filtered on Celite and concentrated under reduced pressure. The resulting residue was purified by column chromatography on silica gel (n-hexane:ethyl acetate = 5:1 → 4:1 → 3:1 → 2:1 → 1:1 → 1:3) to afford a lactam reduction product (395 mg, 58%).
(4) The resulting lactam reduction product (395 mg, 0.733 mmol) was dissolved in 24.4 mL of dichloromethane, trimethylsilylimidazole (537 µL, 3.667 mmol) was added thereto, and the mixture was stirred at room temperature for 3 hours under nitrogen. Water was added to the reaction mixture at 0°C under stirring, the resulting mixture was extracted with dichloromethane, and the organic layer was dried over anhydrous magnesium sulfate. The resulting residue after filtration and concentration under reduced pressure was purified by column chromatography on silica gel (n-hexane:ethyl acetate =15:1 → 10:1) to afford compound (6) (R¹ = 2-naphthylethyl, R² = Me) as an oily product (302.6 mg, 66%).
   (1) The resulting compound (6) (R¹ = 2-naphthylethyl, R² = Me) (91.8 mg, 0.141 mmol) and the compound (7) (R^{3a} = Me) (62.4 mg, 0.1631 mmol) which is obtained by the method described in literature (e.g., J. Med. Chem. 43: 4247-4265 (2000)) were dissolved in a mixed solvent of anhydrous toluene: triethylamine = 1:1 (5 mL). The mixture was added to tetrakis(triphenylphosphine)palladium and the resulting mixture was stirred at 110°C for 2.5 hours under argon. After cooling the reaction solution to room temperature, the solution was concentrated under reduced pressure. The resulting residue was purified by column chromatography on silica gel (n-hexane:ethyl acetate = 100:0 → 10:1 → 8:1) to afford a product (118.4 mg, 92%),
   (2) The resulting product (118.4 mg, 0.1297 mmol) was dissolved in anhydrous tetrahydrofuran (5 mL), triethylaminetris(hydrofluoride) (1.9 mL, 0.0119 mmol) was added thereto at room temperature and the resulting mixture was stirred for 72 hours by increasing the temperature to 40°C. To the reaction solution was added a saturated sodium bicarbonate aqueous solution at 0°C under stirring, and the resulting mixture was extracted with ethyl acetate. The organic layer was washed with saturated brine and dried over anhydrous magnesium sulfate. The resulting residue concentrated under reduced pressure was purified by column chromatography on silica gel (n-hexane:ethyl acetate = 1:2 → 1:3 → 1:4 → 1:5) to afford compound No. 61 (37.7 mg, 48%). The resulting compound No. 61 (37.7 mg) was purified normal phase HPLC (PEGASIL Silica 60-5 column, mobile phase: hexane:ethyl acetate:2-propanol = 100:0:10) to afford compound No. 61a (6.03 mg), compound No. 61b (7.4 mg), compound No. 61c (4.5 mg), and compound No. 61d (5.6 mg), respectively. These four compounds are stereoisomers at the 23-position and the 25-position.
Compound No. 61a:
¹H-NMR (CDCl₃) δ: 7.82-7.31(m, 7H), 6.38 (d, J = 10.9 Hz, 1H), 6.00 (d, J = 11.5 Hz, 1H), 5.29 (s, 1H), 5.01 (s, 1H), 4.32 (bis, 1H), 3.88 (m, 2H), 3.50 (m, 1H), 3.26 (m, 1H), 3.06 (m, 1H), 2.95 (m, 1H), 2.81 (m, 1H) 2.66 (m, 1H), 2.31 (m, 2H), 2.22 (m, 2H), 2.00-0.80 (m, 17H), 1.08 (d, J = 6.9 Hz, 3H), 0.92 (d, J = 6.3 Hz, 3H), 0.53 (s, 3H).
Compound No. 61b:
¹H-NMR (CDCl₃) δ: 7.82.-7.13 1(m, 7H), 6.37 (d, J = 11.0 Hz, 1H), 5.99 (d, J = 11.4 Hz, 1H), 5.27 (s, 1H), 5.00 (s, 1H), 4.31 (brs, 1H),3.82 (m, 2H), 3.48 (m, 1H), 3.32 (m, 2H), 3.02 (m, 2H), 2.82 (m, 1H), 2.66 (dd, J = 13.8, 3.6Hz, 1H), 2.32 (m, 1H), 2.23 (m,2H) 2.00-0.78 (m, 18H), 1.07 (d, J = 6.9 Hz, 3H), 0.80 (d, J = 5.5 Hz, 3H), 0.50 (s, 3H),
Compound No. 61c:
¹H-NMR (CDCl₃) δ: 7.82-7.31(m, 7H), 6.39 (d, J = 11.4 Hz, 1H), 6.01 (d, J = 11.4 Hz, 1H), 5.29 (s, 1H), 5.01 (s, 1H), 4.31 (m, 1H), 4.05 (m, 1H), 3.85 (m, 1H), 3.29 (m, 2H), 3.07 (m, 1H), 2.88 (m, 2H), 2.68 (dd, J = 13.3, 3.7 Hz, 1H), 2.24 (m, 3H), 2.10-0.80 (m, 18H), 1.08 (d, J = 6.9 Hz, 3H), 0.94 (d, J = 6.4 Hz, 3H), 0.55 (s, 3H).
Compopund No. 61d:
¹H-NMR (CDCl₃) δ: 7.82-7.31(m, 7H), 6.37 (d, J = 11.5 Hz, 1H), 6.00 (d, J = 10.9 Hz, 1H), 5.28 (s, 1H), 5.00 (s, 1H), 4.31 (brs, 1H), 4,00 (m, 1H), 3.85 (m, 1H), 3.27 (m, 2H), 3.07 (m, 1H), 2.94 (m, 1H), 2.83 (m, 1H) 2.66 (dd, J = 14.4, 4.6 Hz, 1H), 2.23 (m, 1H), 2.10 (m, 2H), 2.00-0.80 (m, 21H), 0.81 (d, J = 5.7 Hz, 3H), 0.50 (s, 3H).

### Example 8

### Production of 2α-(3-hydroxypropyl)-1α,25-dihydroxyvitamin D₃-26,

### 23-lactam-N-(2-naphthylethyl) (compound No. 62)

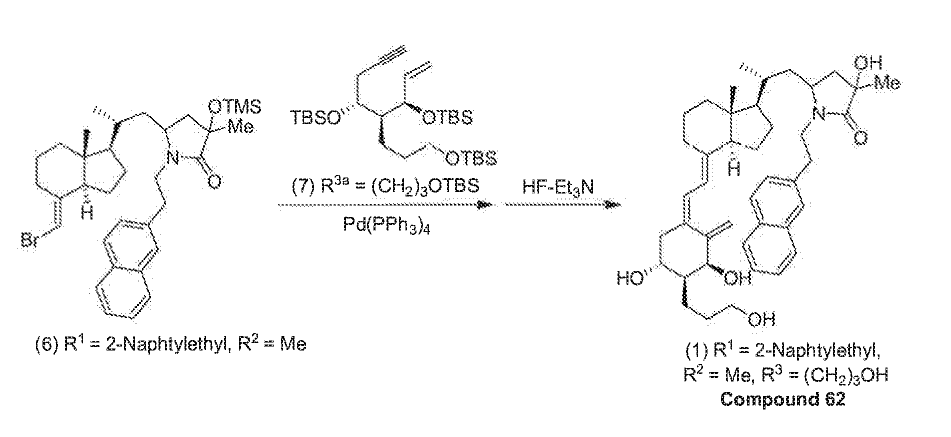

(1) The compound (6) (R¹ = 2-naphthylethyl, R² = Me) (130.4 mg, 0.1998 mmol) obtained in Example 7 and the compound (7) (R³ = -(CH₂)₃OTBS) (125.4 mg, 0.2318 mmol), which is synthesized according to the method of Takayama (Tetrahedron, 60:7951-7961, 2004) obtainable in the literature (e.g., J. Org. Chem. 66: 8760-8771 (2001)), were dissolved in a mixed solvent of anhydrous toluene:triethylamine = 1:1 (5 mL). The mixture was added to tetrakis(triphenylphosphine)palladium and the resulting mixture was stirred at 90°C for 2.5 hours under argon. After cooling the reaction solution to room temperature, the solution was concentrated under reduced pressure. The resulting residue was purified by column chromatography on silica gel (n-hexane:ethyl acetate = 100:0→10:1→8:1) (127.9 mg, 60%).
(2) To a solution of the crude product (127.9 mg, 0.1194 mmol) dissolved in tetrahydrofuran (5 mL) was added triethylaminetris(hydrofluoride) (1.4 mL, 0.0083 mol) at room temperature and the mixture was stirred by increasing the temperature to 40°C for 44 hours. To the reaction solution was added a saturated sodium bicarbonate aqueous solution at 0°C, and the resultant mixture was extracted with ethyl acetate. The organic layer was washed with saturated brine and dried over anhydrous magnesium sulfate. The resulting residue concentrated under reduced pressure was purified by column chromatography on silica gel (n-hexane:ethyl acetate = 1:8 → ethyl acetate → chloroform:methanol = 9:1 → 5:1) to afford compound No. 62 (39.8 mg, 51%). The resulting compound No. 62 (39.8 mg) was purified by normal-phase HPLC (PEGSIL 60-5, mobile phase: hexane:ethyl acetate: 2-propanol = 20:70:10) to afford compound No. 62a (7.8 mg), compound No. 62b (4.0 mg), compound No. 62c (6.6 mg), and compound No. 62d (5.1 mg), respectively. These four compounds are stereoisomers at the 23-position and the 25-position.
Compound No. 62a:
¹H-NMR (CDCl₃) δ: 7.78-7.34 (m, 7H), 6.41 (d, J = 11.0 Hz, 1H), 6.01 (d, J = 11.0 Hz, 1H), 5.31 (s, 1H), 5.02 (brd, 1H), 4.41 (brs, 1H), 3.92 (m, 1H), 3.72 (m, 1H), 3.52 (m, 1H), 3.28 (m, 1H), 3.08 (m, 1H), 2.98 (m, 1H) 2.85 (d, J = 12.8 Hz, 1H), 2.69 (dd, J = 14.0, 4.1 Hz, 1H), 2.32 (m, 2H), 2.00-1.00 (m, 22H), 1.27 (s, 3H), 0.94 (d, J = 7.0 Hz, 3H), 0.53 (s, 3H).
Compound No. 62b:
¹H-NMR (CDCl₃) δ: 7.78-7.34 (m, 7H), 6.39 (d, J = 11.0 Hz, 1H), 5.90 (d, J = 11.0 Hz, 1H), 5.29 (s, 1H), 5.00 (s, 1H), 4.39 (brs, 1H), 4.05 (m, 1H), 3.90 (m, 1H), 3.69 (m, 1H), 3.49 (m, 1H), 3.28 (m, 2H), 3.05 ( m, 1H), 2.92 (m, 1H), 2.82 (m, 1H), 2.67 (m, 1H), 2.38-1.00 (m, 22H), 1.13 (s, 3H), 0.94 (d, J = 7.0 Hz, 3H), 0.54 (s, 3H).
Compound No. 62c:
¹H-NMR (CDCl₃) δ: 7.78-7.34 (m, 7H), 6.38 (d, J = 11.0 Hz, 1H), 5.97 (d, J = 11.0 Hz, 1H), 5.27 (s, 1H), 5.00 (brd, 1H), 4.37 (brs, 1H), 3.90 (m, 1H), 3.80 (m, 1H), 3.70 (m, 2H), 3.47 (m, 1H), 3.33 (m, 1H), 3.02 (m, 2H), 2.81 (d, J = 12.4 Hz, 1H), 2.65 (dd, J = 13.7, 4.5 Hz, 1H), 2.51 (brs, 1H), 2.23 (m, 2H), 1.93 (t, J = 9.0 Hz, 2H), 1.80-1.10 (m, 15H), 1.25 (s, 3H), 0.80 (d, J = 7.0 Hz, 3H), 0.49 (s, 3H).
Compound No. 62d:
¹H-NMR (CDCl₃) δ: 7.78-7.34 (m, 7H), 6.38 (d, J = 11.0 Hz, 1H), 5.98 (d, J = 11.0 Hz, 1H), 5.27 (s, 1H), 4.98 (s, 1H), 4.38 (s, 1H), 4.01 (m, 1H), 3.90 (m, 1H), 3.70 (m, 1H), 3.28 (m, 2H), 3.06 (m, 1H), 2.95 (m, 1H), 2.81 (m, 1H), 2.65 (dd, J = 13.2, 4.1Hz, 1H), 2.24 (dd, J = 13.7, 9.6 Hz, 1H), 2.10 (dd, J = 12.3, 6.0 Hz, 1H), 2.00-1.00 (m, 21H), 1.11 (s, 3H), 0.81 (d, J = 7.0 Hz, 3H), 0.49 (s, 3H).

### Example 9

### Production of 2α-(3-hydroxypropoxy)-1α,25-dihydroxyvitamin D₃-26,

### 23-lactam-N-(2-naphthylethyl) (compound No. 63)

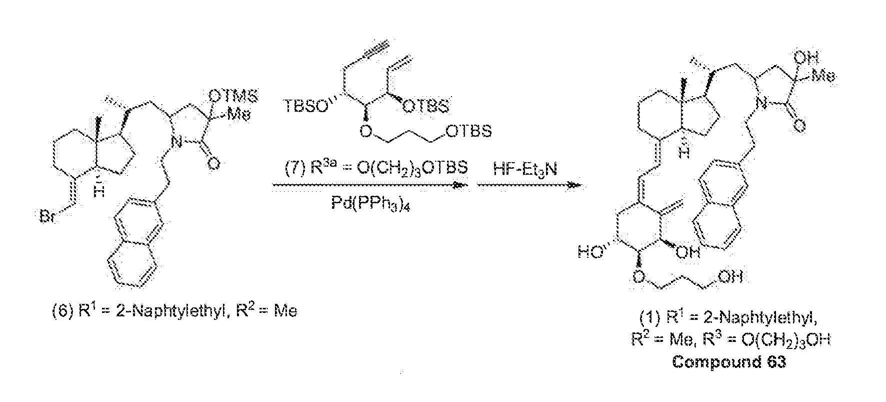

(1) The compound (6) (R¹ = 2-naphthylethyl, R² = Me) (29.8 mg, 0.047 mmol) obtained in the Example 7 and the compound (7) (R ³ = -O(CH₂)₃OTBS) (101.8 mg, 0.1828 mmol) which is obtained by the methods described in literature (e.g., J. Med. Chem., 43:4247-4265 (2000)) were dissolved in a mixed solvent of anhydrous toluene:triethylamine = 1:1 (5 mL). The mixture was added to tetrakis(triphenylphosphine)palladium and the resulted mixture was stirred at 90°C for 2.5 hours under argon. After cooling the reaction solution to room temperature, the solution was concentrated under reduced pressure. The resulting residue was purified by column chromatography on silica gel (n-hexane:ethyl acetate = 100:0 → 20:1 → 10:1) (125.6 mg, 73%).
(2) To a solution of the resulting compound (101.5 mg, 0.0934 mmol) dissolved in tetrahydrofuran (4.5 mL) was added triethylaminetris(hydrofluoride) (1.4 mL, 0.0084 mol) at room temperature and the mixture was stirred by increasing the temperature to 40°C for 72 hours. To the reaction solution was added saturated sodium bicarbonate aqueous solution at 0°C, the mixture was stirred and then extracted with ethyl acetate. The organic layer was washed with saturated brine and dried over anhydrous magnesium sulfate. The resulting residue concentrated under reduced pressure was purified by column chromatography on silica gel (n-hexane:ethyl acetate =1:2 → 1:6 → 1:8 → ethyl acetate → chloroform:methanol = 9:1 → 5:1) to afford compound No.63 (36.3 mg, 58%). The resulting compound No.63 (36.3 mg) was purified by normal-phase HPLC (PEGASIL 60-5, mobile phase: hexane:ethyl acetate:2-propanol = 10:80:10) to afford compound No.63a (8.4 mg), compound No.63b (4.6 mg), compound No.63c (7.2 mg), and compound No.63d (5.12 mg), respectively. These four compounds are stereoisomers at the 23-position and the 25-position.
Compound No. 63a:
¹H-NMR (CDCl₃) δ: 7.7-7.30 (m, 7H), 6.41 (d, J = 11.0 Hz, 1H), 6.01 (d, J = 11.0 Hz, 1H), 5.40 (s, 1H), 5.09 (s, 1H), 4.45 (brs, 1H), 4.11 (m, 1H), 3.81 (m, 5H), 3.50 (m, 1H), 3.39 (dd, J = 3.2, 1.2 Hz, 1H), 3.26 (m, 1H), 3.06 (m, 1H), 2.95 (m, 1H), 2.82 (d, J = 3.4 Hz, 1H), 2.68 (dd, J = 13.7, 4.5 Hz, 1H), 2.56 (brs, 1H), 2.28 (m, 2H), 2.00-1.80 (m, 6H), 1.80-1.00 (m, 10H), 1.42 (s, 3H), 0.90 (d, J = 7.0 Hz, 3H), 0.51 (s, 3H).
Compound No. 63b:
¹H-NMR (CDCl₃) δ: 7.7-7.30 (m, 7H), 6.41 (d, J = 11.0 Hz, 1H), 6.01 (d, J = 11.0 Hz, 1H), 5.40 (s, 1H), 5.10 (s, 1H), 4.45 (brs, 1H), 4.06 (m, 1H), 3.83 (m, 5H), 3.40 (m, 1H), 3.28 (m, 1H), 3.06 (m, 1H), 2.92 (m, 1H), 2.83 (m, 1H), 2.68 (dd, J = 13.7, 4.5 Hz, 1H), 2.32 (m, 1H), 2.26 (m, 1H), 2.16 (m, 1H), 2.00-1.80 (m, 6H), 1.80-1.00 (m, 11H), 1.13 (s, 3H), 0.93 (d, J = 7.0 Hz, 3H), 0.54 (s, 3H).
Compound No. 63c:
¹H-NMR (CDCl₃) δ: 7.7-7.30 (m, 7H), 6.41 (d, J = 11.0 Hz, 1H), 6.01 (d, J = 11.0 Hz, 1H), 5.38 (s, 1H), 5.08 (s, 1H), 4.45 (brs, 1H), 4.08 (m, 1H), 3.81 (m, 5H), 3.50 (m, 1H), 3.38 (dd, J = 3.2, 1.2 Hz, 1H), 3.30 (m, 1H), 3.03 (q, J = 6.8 Hz, 1H), 2.81 (brd, 1H), 2.66 (brd, 1H), 2.60 (brs, 1H), 2.22 (m, 2H), 2.00-1.80 (m, 4H), 1.80-1.00 (m, 15H), 1.42 (s, 3H), 0.80 (d, J = 7.0 Hz, 3H), 0.56 (s, 3H).
Compound No. 63d:
¹H-NMR (CDCl₃) δ: 7.7-7.30 (m, 7H), 6.40 (d, J = 11.0 Hz, 1H), 6.00 (d, J = 11.0 Hz, 1H), 5.38 (s, 1H), 5.08 (s, 1H), 4.44 (brs, 1H), 4.05 (m, 1H), 3.87 (m, 4H), 3.75 (m, 1H), 3.36 (dd, J = 7.8, 3.2 Hz, 1H), 3.28 (m, 1H), 3.05 (m, 1H), 2.94 (m, 1H), 2.83 (m, 1H), 2.67 (dd, J = 13.7, 5.0 Hz, 1H), 2.33 (m, 1H), 2.24 (m, 1H), 2.11 (dd, J = 12.4, 5.9 Hz, 1H), 2.00-1.80 (m, 4H), 1.80-1.00 (m, 3H), 1.10 (s, 3H), 0.80 (d, J = 7.0 Hz, 3H), 0.49 (s, 3H).

### Example 10

### Binding affinity to intracellular vitamin D₃ receptor (VDR) in chick in intestinal mucous membrane

This binding affinity was assayed according to the method described by Ishizuka, et al. (Steroids, 37:33-43, 1982). Specifically, a solution of [26, 27-methyl-³H]1α,25-dihydroxyvitamin D₃ (15,000 dpm, 180 Ci/mmol) in ethanol (10 µL) and a solution of various concentrations of the compounds of the present invention in ethanol (40 µL) were added into a polypropylene tube (12 x 75 mm). The intracellular 1α,25-dihydroxyvitamin D₃ receptor protein in chick intestinal mucous membrane (0.2 mg) and gelatin (1 mg) were dissolved in 1 mL of phosphate buffer (pH 7.4), the solution was added into the tube, and the mixture was allowed to react at 25°C for 1 hour. To the tube, a 40% polyethylene glycol 6000 solution (1mL) was added and the mixture was vigorously stirred. Then the tube was centrifuged at 2260xg at 4 °C for 60 minutes. The bottom of the tube containing precipitate was cut off by a cutter knife and was placed in a liquid scintillation vial, dioxane scintillator (10 mL) was added thereto and the radioactivity of the precipitate was measured by a liquid scintillation counter. The measured values were plotted against the concentration of the test compounds, and IC₅₀ (the concentration of the test compounds at which binding to the receptor was inhibited by 50%) was determined. The ratios of the IC₅₀ of the compounds of the present invention over the IC₅₀ of 1α,25-dihydroxyvitamin D₃ were calculated as the VDR affinity of the compounds of the present invention (%). The results are shown in Table 2.

**Table 2**

| Compound No. | VDR affinity (%) |
|---|---|
| 11a | 50 |
| 12a | 222 |
| 13a | 20 |

### Example 11

### Binding affinity to human vitamin D₃ receptor (VDR)

This binding affinity was assessed by a VDR affinity measurement kit (Polarscreen™ Vitamin D Receptor Competitor assay, Red; Invitrogen Corporation). The compounds of the present invention or 1α,25-dihydroxyvitamin D₃ at various concentrations in DTT-containing buffer (pH 7.5) were mixed with fluorescence-labeled VDR ligand-human VTR complex in DTT-containing buffer (pH 7.5). The degree of polarization of the resulting solution was measured after the incubation of the solution at room temperature. The polarization values were plotted against the concentration of the test compounds, IC₅₀ (the concentration of the test compounds at which the polarized value is reduced to 50%) was determined. The ratios of IC₅₀ of the compounds of the present invention over the IC₅₀ of 1α,25-dihydroxyvitamin D₃ were calculated as the VDR affinity of the compounds of the present invention (%). The results are shown in Table 3.

**Table 3**

| Compound No. | VDR affinity (%) |
|---|---|
| 41a | 31 |
| 41b | 18 |
| 41c | 30 |

The results of Examples 10 and 11 clearly showed that the compounds of the present invention have strong binding affinity to VDR. Accordingly, taking the vitamin D₃ receptor-antagonist activity of the compounds of the present invention to be described later into consideration, it was suggested that these compounds of the present invention are expected to have extremely strong vitamin D₃ receptor-antagonist activity and are useful for treating and/or preventing Paget's disease of bone, hypercalcaemia, osteoporosis, and asthma.

### Example 12

### Vitamin D₃ receptor-antagonist effect in terms of cellular differentiation activity of 1α,25-dihydrovitamin D₃ in HL-60 as a marker

(1) A HL-60 cell line purchased from a cell bank (Japanese Cancer Research Resource Bank, Cell number: JCRB0085) was used. The cells were frozen for storage as a frozen storage stock to prevent cell characteristic changes due to successive cultivations. The cells were defrosted prior to the initiation of the experiment, and those which started successive culturing were used in the experiment. Cells subcultured for one month to a half year were used for the experiment. The successive culturing was carried out by diluting the cells at a ratio of about 1/100 (1-2x10⁴ cells/mL) in a fresh medium after the collection of cells from a cell suspension by centrifugation. RPMI-1640 supplemented with 10% fetal bovine serum was used as the culture medium.
(2) The subcultured cells by the procedure described in (1) were collected by centrifugation and re-suspended in the medium at 5 x 10³ cells/mL, and the cells were plated into a 24-well culture dish at 1 mL/well. To this system, a solution of 1α, 25-dihydroxyvitamin D₃ at a concentration of 1 x 10⁻⁵ M and the compounds of the present invention in a range of 1 x 10⁶ M to 3 x 10⁻³ M in ethanol, respectively, were added at 1 µL per well (the final concentration: 1x10⁻⁸ M for 1α, 25-dihydroxyvitamin D₃, and 1 x 10⁻⁹ M to 3x10⁻⁹ M for the compounds of the present invention). Ethanol was added at 1 µL per well as a control. After the plate was incubated at 37°C for 4 days in the presence of 5% CO₂, the cells were separated and collected by centrifugation.
(3) The induction of nitroblue tetrazolium (hereinafter NBT) reduction activity was used as a marker for cell differentiation-inducing activity of HL-60 cells. The measurement of NBT reduction activity was carried out according to the following procedure. Specifically, cells collected by centrifugation were re-suspended in a fresh medium. Then 0.1% of NBT and 12-O-tetradecanoylphorbor-13-acetate at a final concentration of 100 ng/mL were added to the cell suspension and the mixture was incubated at 37°C for 25 minutes. Then the cytospin smears were prepared and stained with Kernechtrot after air-drying. The ratio of positive cells expressing NBT reduction activity was determined under optical microscopy. Percentage ratios of the ratio of NBT-positive cells in a simultaneous treatment with 1α,25-dihydroxyvitamin D₃ (1 x 10⁻⁸ M) and a compound of the present invention (1 x 10⁻⁹ M and 3 x 10⁻⁶ M) over the ratio of the NBT-positive cells in a single treatment with 1α,25-dihydroxyvitamin D₃ (1 x 10⁻⁸ M) were plotted against the treating concentration of the compounds of the present invention. Then the testing concentration of the compound of the present invention at which the percentage ratio became 50% was calculated as the value of IC₅₀ (nM). The results are shown in Table 4.

**Table 4**

| Compound No. | IC₅₀ (nM) |
|---|---|
| 11a | 16 |
| 12a | 10 |
| 12b | 170 |
| 13a | 52 |

### Example 13

### Vitamin D₃ receptor-antagonist activity in terms of the transcription activity human osteocalcinby 1α,25-dihydroxyvitamin D₃ as a marker

(1) A pGL3 vector (Promega Corp.) was used as a reporter vector. The promoter sequence of the human osteocalcin gene, which was obtained by a known method (Ozono et al., J. Biol. Chem., 265:21881-21888, 1990), was cloned from cDNA acquired from HOS cells (obtained from ATCC) and inserted into the upstream of the luciferase gene on the reporter vector. An expression vector was constructed by inserting a DNA sequence encoding human VDR and human RXR into a pCDNA3 vector (Invitrogen Corp). The HOS cells were incubated in a DMEM medium supplemented with 10% FBS at 37°C in the presence of 5% CO₂, and subcultivated every 2 or 3 days.
(2) Cells being subcultivated were collected by centrifugation and re-suspended in a serum and phenol red-free DMEM medium at 4 x 10⁵ cells/mL. The re-suspension of cells was plated in a 96 well plate at 0.1 mL/well. In this system, various vectors described in (1) were added at 0.05 mL/well with Lipofectamin 2000 (Invitrogen Corp.). After the incubation for 3 hours at 37°C, 1α,25-dihydroxyvitamin D₃ and the compounds of the present invention in various concentrations or TEI-9647 (note) as a control (the final concentration: 1 x 10⁻⁸ M for 1α, 25-dihydroxyvitamin D₃, and 1 x 10⁻⁶ M and 1 x 10⁻¹² M for the compounds of the present invention and TEI-9647) were added to each well. After incubation at 37°C for 24 hours, the medium was removed, the cells were washed with PBS(-) once, and measured for the luciferase activity using a Dual Glo-Luciferase Assay kit (Promega Corp.) by luminometer (Berthold Corp.). The resulting luciferase values were plotted against the concentration of the test compounds and IC₅₀ was determined as the concentration of the test compound at which the luciferase value was inhibited by 50%. Furthermore, to compare the intensities of activity among experiments, the IC₅₀ value of the test compounds with respect to the IC₅₀ value of TEI-9645 in each experiment was calculated as the antagonist specific activity (%) against TEI-9647. The results are shown in Table 5.
(note) TEI-9647: A published VDR antagonist. See reference: J. Biol. Chem., 274:16392-16399,1999.

**Table 5**

| Compound No. | IC₅₀ (nM) | Antagonist specific activity against TEI-9647 (%) |
|---|---|---|
| 11a | 27.5 | 90.5 |
| 12a | 17.4 | 143.1 |
| 13a | 83.3 | 29.9 |
| 41c | 52.7 | 17.8 |
| 42c | 75.0 | 6.1 |
| 43c | 95.8 | 9.8 |

The results in Examples 12 and 13 showed that the compounds of the present invention suppress the cell differentiation-inducing activity induced by 1α,25-dihydroxyvitamin D₃. Accordingly, it is shown that the compounds of the present invention act as antagonists to 1α,25-dihydroxyvitation D₃ receptor.

Since Paget's disease of bone, hypercalcaemia, and asthma are caused by the acceleration of activity of the active form of vitamin D₃, the compounds of the present invention are useful for treating and/or preventing these diseases. In addition, since the PTH level increases in response to the decreasing in vivo concentration of the active form of vitamin D₃, and the PTH has an osteogenesis activity, the compounds of the present invention are useful as a therapeutic agent and/or prophylactic agent for osteoporosis.

### INDUSTRIAL APPLICALIBILITY

The compounds of the present invention can be used as active ingredients of pharmaceutical preparations. Pharmaceutical compositions comprising the compounds of the present invention as active ingredients are useful for therapeutic and/or prophylactic agents for one or plurality of diseases selected from the group consisting of Paget's disease of bone, hypercalcaemia, osteoporosis and asthma.

## Claims

1. A vitamin D₃ derivative represented by Formula (1):
wherein R¹ represents a C₁-C₁₀ alkyl group or a C₇-C₁₅ aralkyl group whose aromatic ring is optionally substituted with a C₁-C₆ alkyl group, a C₁-C₆ alkoxy group, a hydroxyl group, a halogen atom or a trifluoromethyl group; R² represents a C₁-C₆ alkyl group; R³ represents a C₁-C₆ alkyl group optionally substituted with a hydroxyl group or a C₁-C₆ alkoxy group optionally substituted by a hydroxyl group or a pharmaceutically acceptable solvate thereof.

2. The vitamin D₃ derivative or a pharmaceutically acceptable solvate thereof according to claim 1, wherein R¹ is a phenethyl group, a naphthylmethyl group, a dimethoxybenzyl group, a dicthoxybenzyl group, a dipropoxybenzyl group, or a naphthylethyl group.

3. The vitamin D₃ derivative or a pharmaceutically acceptable solvate thereof according to claim 1, wherein R¹ is a phenethyl group, a 3,5-diethoxybenzyl group, or a 2-naphthylethyl group.

4. The vitamin D₃ derivative or a pharmaceutically acceptable solvate thereof according to any of claims 1 to 3, wherein R² is a methyl group.

5. The vitamin D₃ derivative or a pharmaceutically acceptable solvate thereof according to any of claims 1 to 4, wherein is a methyl group, a 3-hydroxypropyl group, or a 3-hydroxypropoxy group.

6. The vitamin D₃ derivative or a pharmaceutically acceptable solvates thereof according to claim 1, wherein R¹ is a phenethyl group, a 3,5-diethoxybenzyl group, or a 2-nathtylethyl group; R² is a methyl group; and R³ is a methyl group, a 3-hydroxypropyl group or a 3-hydroxypropoxy group.

7. The vitamin D₃ derivative or a pharmaceutically acceptable solvates thereof according to claim 1, wherein R¹ is a phenethyl group; R² is a methyl group; and R³ is a methyl group.

8. The vitamin D₃ derivative or a pharmaceutically acceptable solvates thereof according to claim 1, wherein R¹ is a phenethyl group; R² is a methyl group; and R³ is a 3-hydroxypropyl group.

9. The vitamin D₃ derivative or a pharmaceutically acceptable solvate thereof according to claim 1, wherein R¹ is a phenethyl group; R² is a methyl group; and R³ is a 3-hydroxypropoxy group.

10. The vitamin D₃ derivative or a pharmaceutically acceptable solvate thereof according to claim 1, wherein R¹ is a 3,5-diethoxybenzyl group; R² is a methyl group; and R³ is a methyl group.

11. The vitamin D₃ derivative or a pharmaceutically acceptable solvate thereof according to claim 1, wherein R¹ is a 3,5-diethoxybenzyl group; R² is a methyl group; and R³ is a 3-hydroxypropyl group.

12. The vitamin D₃ derivative or a pharmaceutically acceptable solvate thereof according to claim 1, wherein R¹ is a 3,5-diethoxybenzyl group; R² is a methyl group; and R³ is a 3-hydroxypropoxy group.

13. The vitamin D₃ derivative or a pharmaceutically acceptable solvate thereof according to claim 1, wherein R¹ is a 2-naphthylethyl group; R² is a methyl group; and R³ is a methyl group.

14. The vitamin D₃ derivative or a pharmaceutically acceptable solvate thereof according to claim 1, wherein R¹ is a 2-naphthylethyl group; R² is a methyl group; and R³ is a 3-hydroxypropyl group.

15. The vitamin D₃ derivative or a pharmaceutically acceptable solvate thereof according to claim 1, wherein R¹ is a 2-naphthylethyl group; R² is a methyl group; and R³ is a 3-hydroxypropoxy group.

16. The vitamin D₃ derivative or a pharmaceutically acceptable solvate thereof according to any of claims 1 to 15, wherein the configurations at 23-position and 25-position in the above Formula (1) are 23(S) and 25 (S), respectively.

17. The vitamin D₃ derivative or a pharmaceutically acceptable solvate thereof according to any of claims 1 to 16 having a vitamin D₃ antagonist activity

18. A therapeutic and/or prophylactic agent for one or plurality of diseases selected from the group consisting of hypercalcaemia, Paget's disease of bone, osteoporosis and asthma, comprising the vitamin D₃ derivative or a pharmaceutically acceptable solvate thereof according to any of claims 1 to 17 as an active ingredient.

19. A therapeutic and/or prophylactic agent for hypercaleaemia, comprising the vitamin D₃ derivative or a pharmaceutically acceptable solvate thereof according to any of claims 1 to 17 as an active ingredient.

20. A therapeutic and/or prophylactic agent for Paget's disease of bone, comprising the vitamin D₃ derivative or a pharmaceutically acceptable solvate thereof according to any of claims 1 to 17 as an active ingredient.

21. An agent for treating and/or preventing osteoporosis, comprising the vitamin D₃ derivative or a pharmaceutically acceptable solvate thereof according to any of claims 1 to 17 as an active ingrédient.

22. An agent for treating and/or preventing asthma, comprising the vitamin D₃ derivative or a pharmaceutically acceptable solvate thereof according to any of claim1 to 17 as an active ingredient.
